(19) Europäisches Patentamt
European Patent Office
Office européen des brevets

(11) **EP 3 528 932 B1**

(12) **EUROPÄISCHE PATENTSCHRIFT**

(45) Veröffentlichungstag und Bekanntmachung des Hinweises auf die Patenterteilung:
**29.11.2023  Patentblatt 2023/48**

(21) Anmeldenummer: **17787409.6**

(22) Anmeldetag: **23.10.2017**

(51) Internationale Patentklassifikation (IPC):
**B01D 63/02** *(2006.01)*     **B01D 65/10** *(2006.01)*
**A61M 1/16** *(2006.01)*

(52) Gemeinsame Patentklassifikation (CPC):
**B01D 63/021; A61M 1/16; A61M 1/1621;**
**B01D 65/10;** A61M 2205/3379; A61M 2207/00;
B01D 2313/04; B01D 2323/42

(86) Internationale Anmeldenummer:
**PCT/EP2017/076959**

(87) Internationale Veröffentlichungsnummer:
**WO 2018/077781 (03.05.2018 Gazette 2018/18)**

(54) **VERFAHREN UND VORRICHTUNG ZUR BESTIMMUNG EINER PERMEATIONSEIGENSCHAFT VON HOHLFASERMEMBRANBÜNDELN**

METHOD AND SYSTEM FOR DETERMINING A PERMEATION PROPERTY OF HOLLOW FIBRE MEMBRANE BUNDLES

PROCÉDÉ ET SYSTÈME POUR DÉTERMINER UNE PROPRIÉTÉ DE PERMÉATION DE FAISCEAUX DE MEMBRANES À FIBRES CREUSES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.10.2016  DE 102016012722**

(43) Veröffentlichungstag der Anmeldung:
**28.08.2019  Patentblatt 2019/35**

(73) Patentinhaber: **Fresenius Medical Care Deutschland GmbH**
**61352 Bad Homburg (DE)**

(72) Erfinder:
• **GRAUSAM, Elmar**
  **Bad Homburg 61352 (DE)**
• **FEHR, Ingo**
  **Bad Homburg 61352 (DE)**

(74) Vertreter: **Ricker, Mathias**
**Wallinger Ricker Schlotter Tostmann**
**Patent- und Rechtsanwälte Partnerschaft mbB**
**Zweibrückenstraße 5-7**
**80331 München (DE)**

(56) Entgegenhaltungen:
EP-A1- 0 958 852     EP-A1- 2 418 012
WO-A1-2015/014934    DE-A1-102013 203 082
JP-A- H04 150 926    JP-A- 2007 216 175
US-A- 4 678 573

**Beschreibung**

**Thema der Erfindung**

Die Erfindung betrifft ein Verfahren zur Bestimmung einer Permeationseigenschaft von Hohlfasermembranen in einem Hohlfasermembranbündel. Insbesondere betrifft die Erfindung ein Verfahren mit dem aus einer ermittelten Permeationseigenschaft der Ultrafiltrationskoeffizient von Hohlfasermembranen in einem Hohlfasermembranbündel bestimmt werden kann.

Darüber hinaus betrifft die Erfindung eine Vorrichtung zur Durchführung eines Verfahrens zur Bestimmung einer Permeationseigenschaften, insbesondere der Bestimmung der Ultrafiltrationsrate, von Hohlfasermembranen in einem Hohlfasermembranbündel.

Weiterhin betrifft die Erfindung ein Verfahren zur Herstellung von Hohlfasermembran-Filtermodulen aus Hohlfasermembranen mit wenigstens einer vorbestimmten Permeationseigenschaften, insbesondere einer vorbestimmten Ultrafiltrationsrate.

**II. Hintergrund der Erfindung**

Hohlfasermembranen finden in der Aufreinigung von Flüssigkeiten weiträumig Verwendung. Insbesondere werden Hohlfasermembranen in der Medizintechnik für Wasseraufbereitung und Blutreinigung insbesondere in der Dialyse von nierenkranken Patienten eingesetzt. Entsprechende Hohlfasermembranen werden in Hohlfasermembranbündeln in Filtermodulen verbaut. Die Herstellung solcher Filtermodule zur Blutreinigung erfolgt mittlerweile im Massenproduktionsmaßstab.

Der Bau von Hohlfasermembran-Filtermodulen und die Herstellung von Hohlfasermembranen für die Dialyse ist im Stand der Technik bekannt (Uhlenbusch-Körwer, Bonnie- Schorn, Grassmann, Vienken, "Understanding Membranes and Dialysers" Publisher: Pabst Science Publishers, 2004).

Die für die Blutreinigung verwendeten Hohlfasermembranen bestehen häufig aus Polysulfon (PSU) und Polyvinylpyrrolidon (PVP) und werden in der Regel in einem sogenannten "dry-wet" Spinnverfahren hergestellt. In solchen Verfahren wird eine Spinnlösung, die die Polymere PSU und PVP und ein Lösungsmittel enthält durch eine Ringspaltdüse zu einem hohlen Spinnfaden extrudiert. Der Spinnfaden wird zunächst senkrecht durch einen Luftraum, den sog. Fällspalt geführt. In das Lumen des Spinnfadens wird mit der Extrusion des Spinnfadens gleichzeitig ein Koagulationsmedium extrudiert, so dass mit der Extrusion des Spinnfadens einen Koagulationsprozess beginnt. Bei der Koagulation kommt es zu einer Phaseninversion, so dass sich innerhalb des Spinnfadens sogenannte Sol und Gel Phasen bilden. Der Spinnfaden wird nach Durchlaufen des Fällspalts in ein Fällbad eingeleitet, in dem der Faden vollständig zur Fällung gebracht wird und sich die feste Hohlfasermembranstruktur ausbildet. Anschließend durchläuft die erhaltene Hohlfasermembran mehrere Spülbäder und Trocknungszonen. Je nach voreingestellten Spinnbedingungen erhält man Hohlfasermembranen mit unterschiedlicher Porenstruktur und -eigenschaften. Der Begriff "PSU" wird im Rahmen dieser Anmeldung als generischer Begriff für alle Polymere verstanden, die Sulfongruppen enthalten wie beispielsweise auch Polyethersulfon und Polyphenylsulfon und Copolymere umfassend diese Polymere.

Im Allgemeinen wird in einer Produktionsanlage eine Vielzahl von Hohlfasermembranen gleichzeitig und parallel extrudiert, so dass die nach Durchlaufen der Produktionsanlage erhaltenen Hohlfasermembranfasern als Fadenschar oder Faserbündel zusammengefasst werden können und von einer Haspel aufgenommen werden. Entsprechende Verfahren sind im Stand der Technik bekannt und z.B. in der DE 10 2006 057 101 A1 beschrieben.

Der Herstellprozess von Hohlfasermembranen ist ein kontinuierlicher Prozess, der in den Produktionsstätten im Dreischicht Betrieb betrieben wird. Es ist daher fortlaufend notwendig die Qualität der hergestellten Fasern zu überwachen, um größere Fehlproduktionen zu vermeiden. Daher wird laufend geprüft, ob die Eigenschaften der hergestellten Hohlfasermembran im spezifizierten Bereich liegen und der Produktionsprozess nach den voreingestellten Bedingungen abläuft.

Hohlfasermembranen werden insbesondere über ihre Permeationseigenschaften charakterisiert. Anhand der Permeationseigenschaft einer Hohlfasermembran ist zu entscheiden, ob die Hohlfasermembran für bestimmte Trennprozesse, z.B. in der Dialyse von nierenkranken Patienten, verwendet werden kann. Zur Ermittlung einer Permeationseigenschaft von Hohlfasermembranen war es bislang notwendig aus den hergestellten Hohlfasermembranbündeln Filtermodule zu bauen, an denen dann erst die Trenneigenschaften der Membran festgestellt werden konnten.

Im Filterbau müssen die Hohlfasermembranbündel in ein Filtergehäuse eingeformt werden und endseitig vergossen werden. Der Verguss erfolgt mit aushärtbaren Harzmassen, insbesondere mit Polyurethan. Der Verguss selbst und die Aushärtung sind zeitaufwendige Verfahrensschritte, so dass das Filterbau und die folgenden Analysen an den Filtern in den Produktionslaboren mehrere Stunden in Anspruch nehmen. Während dieser Zeit läuft die Produktion der Hohlfasermembranen weiter. Im Zweifelsfall kann es vorkommen, dass die Analysenergebnisse nach einigen Stunden ergeben, dass die Spezifikationen der hergestellten Hohlfasermembran nicht mehr eingehalten werden, so dass das Produktionsergebnis mehrerer Stunden verworfen werden muss.

Ergeben die Analyseergebnisse dagegen, dass die hergestellten Hohlfasermembranen die Spezifikationen erfüllen, so kann dagegen das entsprechende Produktionsergebnis an Hohlfasermembranen weiter zu den entsprechenden Filtermodulen verarbeitet werden.

Wie vorab erwähnt, werden derartige Hohlfasermembran-Filtermodule für die Blutreinigung von nierenkranken Patients verwendet. Die Blutreinigung nierenkranker Patienten basiert auf dem Prinzip des transmembranen Stoffaustausches. Dabei wird Blut an der Innenseite der Hohlfasermembranen geführt, während auf der Außenseite der Hohlfasermembranen eine entsprechende Dialysierflüssigkeit geführt wird. Die Strömungsrichtung der Flüssigkeiten ist dabei entgegengesetzt, so dass eine Filtration im Gegenstromprinzip einsetzt.

Gemäß der Filtrationsbedingungen in Blutbehandlungen von nierenkranken Patienten werden aus dem Patientenblut unerwünschte Metaboliten wie z.B. Harnstoff, Kreatinin, Phosphat und schädliche Plasmaproteine entfern, indem diese durch die Membranwand permeieren und gegebenenfalls von der Dialysierflüssigkeit aufgenommen werden. Ebenso können auch gelöste Bestandteile der Dialysierflüssigkeit durch die Membranwand auf die Blutseite permeieren. Die Dialysierflüssigkeit wird als wässrige physiologische Lösung bereitgestellt, die z.B. Elektrolyte (z.B. $Na^+$, $K^+$, $Mg^{2+}$, $Ca^{2+}$, $Cl^-$), Glukose und einem Puffer (z.B. NaHCO3) enthalten.

Grundsätzlich kann der transmembrane Stoffaustausch in dem geschilderten Prinzip der Blutreinigung auf zwei Arten erfolgen, durch konvektiven Stoffaustausch oder diffusiven Stoffaustausch.

Beim konvektiven Stofftransport permeiert Flüssigkeit und darin befindliche Substanzen gemäß eines transmembranen Druckgradienten durch die Membran. Beim diffusiven Stofftransport erfolgt die Permeation von Substanzen durch die Membran aufgrund der molekularen Eigenbewegung der gelösten Substanzen und einem Konzentrationsgefälle, das über die Membranwand hinweg besteht.

Je nach Art der Hohlfasermembran, bzw. der Porengröße der Hohlfasermembran und der eingestellten Strömungsrate von Blut und Dialysat kann der Filtrationsprozess nach der einen oder anderen Art erfolgen. Werden z.B. für die Blutbehandlung in einem Hämofiltrations- oder Hämodiafiltrationsverfahren Hohlfasermembranen mit großen relativ großen Poren verwendet und/oder werden hohe Strömungsraten der beiden Flüssigkeitsströme, Blut und Dialysat, voreingestellt, so erfolgt wenigstens ein Teil der Filtration konvektiv.

Erfolgt die Blutbehandlung dagegen im Rahmen einer einfachen Dialyse, so werden Membranen mit kleineren Poren verwendet und der Strofftransport erfolgt im Wesentlichen diffusiv. Unter diesen Bedingungen permeieren vor allem nur niedrigmolekulare Substanzen, wie zum Bespiel Harnstoff, Elektrolyte, Vitamin B12, Kreatinin und Phosphat.

Die Abtrennung von schädlichen Plasmaproteinen im Blut von nierenkranken Patienten, kann dagegen nur in therapeutischen Verfahren erreicht werden, in denen ein konvektiver Stofftransport ermöglicht wird. Solche Plasmaproteine liegen im sogenannten Mittelmolekularbereich der Plasmaproteine. Bekannte Proteine im Mittelmolekularbereich sind β-2 Mikroglobulin oder Interleukin-6.

Abhängig von der Porengröße stellt der Ultrafiltrationskoeffizient eine weitere Permeationseigenschaft von Hohlfasermembranen dar. Der Ultrafiltrationskoeffizient gibt in einem vorgegebenen Filtrationsaufbau die Durchlässigkeit der Hohlfasermembran für eine Flüssigkeit, in der Regel Wasser, pro Zeiteinheit und Druckdifferenz an. Methoden zur Bestimmung des Ultrafiltrationskoeffizienten von Hohlfasermembranen sind im Stand der Technik bekannt. Diesbezüglich wird auf die Norm DIN/ EN/ ISO 8637:2014 verwiesen. In der Norm wird die Verwendung von Blut als Prüfflüssigkeit beschrieben. In Abwandlung der Norm wird jedoch häufig Wasser oder Blutplasma als Prüfflüssigkeit verwendet. Insbesondere Wasser ist weit verfügbar und die Messung wird erheblich vereinfacht. Man spricht daher auch von dem "wässrigen" Ultrafiltrationskoeffizienten.

Eine ungewollte Veränderung eines Parameters während der Herstellung von Hohlfasermembranen kann sich auf den Phaseninversionsprozess auswirken. Demzufolge kann sich die mittlere Porengröße, oder die Porenanzahl pro Membranfläche ändern, was direkt eine Auswirkung auf die feststellbare Ultrafiltration hat. In der Produktion von Hohlfasermembranen ist daher insbesondere die Spezifikation zur Ultrafiltrationsrate einer Hohlfasermembran wichtig um den Produktionsprozess überwachen zu können.

Aus dem Stand der Technik ist das in der WO 2013/034611 beschriebene Verfahren zur Bestimmung einer Permeationseigenschaft von Hohlfasermembranen bekannt. Auf S. 18 der WO 2013/034611 A1 wird ein Verfahren zur Bestimmung der hydraulischen Permeabilität an einem endvergossenem Hohlfasermembranbündel beschrieben.

JPH04-150926 offenbart ein Hohlfaserbündel in einem Gehäuse, wobei die Hohlfasern einseitig verschlossen sind. Gas kann in das Bündel eingeführt werden und wieder austreten. Gemäß der Beschreibung wird ein Permeationstest durchgeführt.

EP 0 958 852 A1 offenbart ein Testverfahren und eine Vorrichtung zum Testen eines Hohlfaserbündels, wobei die Fasern einseitig verschlossen, z.B. in Form eines Ultrafiltrationsmoduls. Die Integrität der Fasern wird mittels Wasser- und Gasbeaufschlagung untersucht.

JP 2007216175, US 4,678,573, WO 2015/014934 A1, offenbart Hohlfasermembranbündel, die einen komprimierten Teil aufweisen.

DE 10 2013 203082 A1 offenbart Prüf- und Trocknungseinrichtungen zur Funktionsprüfung von Dialysatoren mit Hilfe eines Luftstroms.

DE 2418 012 A1 discloses a process for testing a filter comprising two compartments separated by a porous membrane wherein the membrane is initially wetted by a testing liquid by establishing a pressure gradient between the compartments and monitoring the pressure gradient between the compartments or measuring gas flow through the membrane.

Es hat sich gezeigt, dass bisherige Methoden zur Qualitätsüberwachung in der Produktion von Hohlfasermembranen verbesserungswürdig sind. Es war daher ein Ziel der Erfindung ein vereinfachtes Verfahren zur Bestimmung einer Permeationseigenschaft, insbesondere der Ultrafiltrationsrate von Hohlfasermembranen bereitzustellen

Ein weiteres Ziel der Erfindung war es, eine Vorrichtung bereitzustellen unter deren Verwendung Hohlfasermembranbündel untersucht werden können und die Ultrafiltrationsrate der Hohlfasermembranen bestimmt werden kann.

Ein weiteres Ziel der Erfindung war es zudem ein Herstellverfahren von Hohlfasermembranen bereitzustellen, das es ermöglicht Hohlfasermembranen innerhalb eines Produktionsverfahrens so herzustellen, dass die Spezifikationen einer Permeationseigenschaft der Hohlfasermembranen, insbesondere der Ultrafiltrationsrate, eingehalten werden kann.

**Zusammenfassung der Erfindung**

Gemäß einem ersten Aspekt der Erfindung wurde überraschenderweise gefunden, dass die genannten Probleme zur Bestimmung einer Permeationseigenschaft durch Bereitstellung eines Verfahrens nach Anspruch 1 gelöst werden können. Die Ansprüche 2 bis 11 stellen bevorzugte Ausführungen der Erfindung des ersten Aspekts da.

Weiterhin wurde in einem zweiten Aspekt der Erfindung gefunden, dass die beschriebenen Probleme durch eine Vorrichtung nach Anspruch 12 gelöst werden können. Anspruch 13 stellt eine bevorzugte Ausführungsform da.

In einem dritten Aspekt betrifft die Erfindung ein Verfahren zur Herstellung von Hohlfasermembran-Filtermodulen nach den Ansprüchen 14 und 15 unter Verwendung eines Verfahrens gemäß der Ansprüche 1 bis 11.

**Detaillierte Beschreibung der Erfindung**

Überraschenderweise wurde in einem ersten Aspekt der Erfindung gefunden, dass sich die vorab genannte Aufgabe durch ein Verfahren zur Bestimmung der Ultrafiltrationsrate und/ oder des Ultrafiltrationskoeffizienten, von Hohlfasermembranen gelöst wird, wobei die Ultrafiltrationsrate die Menge der permeierten Flüssigkeit pro Zeiteinheit ist und der Ultrafiltrationskoeffizient der Ultrafiltrationsrate bezogen auf die transmembrane Druckdifferenz entspricht. Das Verfahren weist die folgenden Schritte auf

(a) Bereitstellen eines Hohlfasermembranbündels, aufweisend eine Vielzahl von Hohlfasermembranen mit einem ersten Ende und einem zweiten Ende, wobei die Lumen der Hohlfasermembranen an dem ersten Ende des Hohlfasermembranbündels endseitig offen, insbesondere flüssigkeitsdurchlässig und an dem zweiten Ende des Hohlfasermembranbündels endseitig verschlossen, insbesondere flüssigkeitsdicht ausgestaltet sind,

(b) Bereitstellen eines Gehäuses zur Aufnahme des Hohlfasermembranbündels aufweisend ein erstes Ende und ein zweites Ende, wobei das erste Ende mindestens einen Flüssigkeitseinlass aufweist,

(c) Einbringen des Hohlfaserbündels in das Gehäuse wobei das erste Ende des Hohlfaserbündels mit dem endseitig offenen Lumen der Hohlfasermembranen zu dem zumindest einen Flüssigkeitseinlass am ersten Ende des Gehäuses ausgerichtet ist,

(d) Bestimmen einer Permeationseigenschaft der Hohlfasermembranen,

(e) Bereitstellen von zumindest einer Kompressionsvorrichtung (204, 340, 450, 451, 460, 461) zum komprimieren des Hohlfasermembranbündels in zumindest einem Teilbereich (222, 322) des Hohlfasermembranbündels, insbesondere des Teilbereichs des Hohlfasermembranbündels, welcher sich an das erste Ende des Hohlfasermembranbündels anschließt, um die Packungsdichte der Hohlfasermembranen in dem zumindest einen Teilbereich des Hohlfasermembranbündels zu erhöhen

(f) Bereitstellen von zumindest einem Flüssigkeitsauslass (204, 340, 440),

(g) Bereitstellen von zumindest einer Prüfflüssigkeit,

(h) Zuströmen der zumindest einen Prüfflüssigkeit durch den wenigstens einen Flüssigkeitseinlass (212, 312, 412) am ersten Ende des Gehäuses (101, 201, 301, 401) ins Innere des Gehäuses und/oder des Hohlfasermembranbündels und/ oder der Hohlfasermembranen (420,421),

(i) Komprimieren des Hohlfasermembranbündels in zumindest einem Teilbereich des Hohlfasermembranbündels (222, 322), insbesondere des Teilbereichs des Hohlfasermembranbündels, welcher sich an das erste Ende des Hohlfasermembranbündels anschließt mittels der zumindest einen Kompressionsvorrichtung (203, 340, 450, 451, 460, 461), um die Packungsdichte der Hohlfasermembranen in dem zumindest einen Teilbereich des Hohlfasermembranbündels zu erhöhen,

wobei in Schritt (d) die Ultrafiltrationsrate und/ oder der auf die transmembrane Druckdifferenz bezogene Ultrafiltrationskoeffizient, der Hohlfasermembranen, durch Aufnehmen wenigstens eines Messwertes, insbesondere durch Messung der austretenden Menge der zumindest einen Prüfflüssigkeit, an zumindest einem Flüssigkeitsauslass (204, 340, 440) bestimmt wird, dadurch gekennzeichnet, dass der komprimierte Teilbereich des Hohlfasermembranbündels (222, 322) so komprimiert ist, dass ein Flüssigkeitsstrom der zuströmenden Prüfflüssigkeit zwischen den Hohlfasermembranen im Wesentlichen unterbunden wird und die Enden des Hohlfasermembranbündels nicht in einem Gießharz vergossen sind.

Es hat sich gezeigt, dass es mit Hilfe der angegebenen Verfahrensschritte möglich ist, Permeationseigenschaften von Hohlfasermembranen zu bestimmen, ohne dass zuvor Hohlfasermembran- Filtermodule aus den Hohlfasermembranbündeln hergestellt werden müssen. Es entfällt so der Aufwand, der für die Herstellung der Prüffiltermodule bereitzustellen ist. Die Bestimmung der Permeationseigenschaft der Hohlfasermembran wird durchgeführt, ohne vorher einen Verguss des Hohlfasermembranbündels durchzuführen. •

Es hat sich gezeigt, dass die Aufnahme des wenigstens einen Messwert, insbesondere die Aufnahme des Wertes der austretenden Menge der zumindest einen Prüfflüssigkeit, an dem zumindest einem Flüssigkeitsauslass, repräsentativ für eine Permeationseigenschaft der untersuchten Hohlfasermembran im Hohlfasermembranbündel ist. Gemäß dem Verfahren kann die Permeationseigenschaft der Hohlfasermembranen bestimmt werden, ohne dass zuvor Holfasermembran-Filtermodule aus den Hohlfasermembranbündeln hergestellt werden müssen. Es entfällt so der Aufwand der für die Herstellung der Prüffiltermodule bereitzustellen ist.

Zur Herstellung von aus dem Stand der Technik bekannten Hohlfasermembran-Filtermodulen werden entsprechende Hohlfasermembranbündel in ein Gehäuse eines Filtermoduls eingeformt und endseitig mit einem Gießharz im Gehäuse vergossen. Der Zwischenraum zwischen den Fasern wird dabei mit dem Gießharz befüllt, so dass endseitig keine Flüssigkeit den Zwischenraum zwischen den Fasern durchdringen kann. Zunächst sind auch die Lumen der Hohlfasermembranen an den Enden verschlossen. Die Lumen der Hohlfasermembranen werden wieder freigelegt, indem endseitig ein Teil des Vergusses abgetrennt wird. Das erfindungsgemäße Verfahren hat den Vorteil, dass der auf den endseitigen Verguss des Hohlfasermembranbündels verzichtet werden kann. Im Rahmen der vorliegenden Erfindung müssen die Hohlfasermembranen lediglich an einem Ende verschlossen werden.

Weiterhin ist ein Effekt der Erfindung gemäß des ersten Aspekts darin zu sehen, dass die Hohlfasermembranen innerhalb kurzer Zeit, d.h. innerhalb weniger Minuten nach ihrer Herstellung hinsichtlich ihrer Permeationseigenschaft untersucht werden können. Untersuchungen nach bisherigen Methoden, die den Bau kompletter Testfiltermodule verlangen, nehmen wenigstens eine Zeitdauer von 3 Stunden in Anspruch. Während dieses Zeitraums kann es bereits zu einer großen Ausschussproduktion kommen, die sich mit dem erfindungsgemäßen Verfahren vermeiden lassen. Somit können z.B. während des Produktionsprozesses von Hohlfasermembranen in kurzer Zeit Rückschlüsse auf die Qualität des Produktionsprozesses gemacht werden und gegebenenfalls entsprechende Maßnahmen ergriffen um den Produktionsprozess ohne Zeitverzug zu steuern. Das erfindungsgemäße Verfahren hat somit den Vorteil schnell und zuverlässig Ergebnisse zur Steuerung der Produktion von Hohlfasermembranen zu liefern. Dadurch können größere Fehlproduktionen vermieden werden

In einem ersten Schritt des erfindungsgemäßen Verfahrens wird ein Hohlfasermembranbündel bereitgestellt, aufweisend eine Vielzahl von Hohlfasermembranen mit einem ersten Ende und einem zweiten Ende, wobei die Öffnungen der Lumen der Hohlfasermembranen an dem ersten Ende des Hohlfasermembranbündels endseitig offen, insbesondere flüssigkeitsdurchlässig, und an dem zweiten Ende des Hohlfasermembranbündels endseitig verschlossen, insbesondere flüssigkeitsdicht, ausgestaltet sind.

Unter dem Begriff "*Hohlfasermembranbündel*" ist im Sinne der vorliegenden Anmeldung ein Bündel aus einer Vielzahl von Hohlfasermembranen zu verstehen. Eine "Hohlfasermembran" weist eine kapillarartige Struktur auf. Insbesondere ist die Hohlfasermembran eine Membran in Form eines hohlen Fadens, der aus einem porösen Material besteht und einen im Wesentlichen kreisförmigen Durchmesser aufweist. Die Wandstärken von solchen Hohlfasermembranen, die für die Dialyse vorgesehen sind können, je nach Membranmaterial 10 bis 100 $\mu$m betragen. Gängige Lumendurchmesser derartiger Hohlfasermembranen liegen zwischen 150$\mu$m bis 250$\mu$m, insbesondere zwischen 180$\mu$m und 220$\mu$m, die Faserlänge liegt im Bereich von 150$\mu$m bis 300 mm, insbesondere zwischen 250mm und 300mm. Der Hohlraum von Hohlfasermembranen kann dabei von Flüssigkeiten durchspült werden. Insbesondere ist vorgesehen an Hohlfasermembranen Stofftrennung vorzunehmen, die durch Ausnutzung einer Permeation von Stoffen durch einen transmembranen Stoffübergang von der Außenseite zum inneren Hohlraum, oder vom inneren Hohlraum zur Außenseite der Hohlfasermembran erfolgen. Derartige Hohlfasermembranen finden typischerweise in der therapeutischen Blutbehandlung Anwendung.

Das Material der Hohlfasermembranen kann ausgewählt werden aus Polymeren, vorzugsweise aus Polysulfon, Polyethersufon, Polyvinylpyrollidon, Polypropylen, Polyacrylnitril, Polyamid, Polyethylenether, Zellulose, Zelluloseregenerat, Celluloseacetat oder Mischungen davon. Besonders bevorzugt sind Membranen, die ein hydrophobes Polymermaterial, wie z.B. Polysulfon oder Polyethersulfon und ein hydrophiles Polymermaterial, wie z.B. Polyvinylpyrrolidon aufweisen, insbesondere daraus bestehen. Die Hohlfasermembran sind derart ausgestaltet, dass das Membranmaterial eine Vielzahl von Poren aufweist, welche dazu dienen einen Stoffaustausch zwischen dem Inneren der Hohlfasermembran und der die Hohlfasermembran umgebenden Umgebung ermöglicht wird und insbesondere entsprechend der Größe der Partikel der jeweiligen Stoffe, einen selektiven Stoffaustausch ermöglicht wird. Das hohle Innere der Hohlfasermembran wird "Lumen" genannt.

Im Sinne der vorliegenden Anmeldung ist unter dem Begriff "Lumen" ein zusammenhängender Hohlraum, der sich im Inneren von Hohlfasermembran der Länge nach vom ersten Ende bis zum zweiten Ende der Hohlfasermembran erstreckt, zu verstehen. Das Lumen von Hohlfasermembranen ist umgeben von der porösen Membranwand, so dass Flüssigkeiten und/oder Gase, die durch das Innere des Lumens geleitet werden, entlang der Hohlfasermembran mit der Membranwand in Stoffaustauschkontakt stehen und ein transmembraner Stoffübergang beobachtet werden kann. Die Öffnungen der Lumen an den Faserenden bilden dabei Zugänge um Stoffe, insbesondere Flüssigkeiten und/ oder Gase in das Faserinnere hinein beziehungsweise wieder hinaus strömen zu lassen.

Beim Zusammenschluss mehrerer Hohlfasermembranen zu einem Hohlfasermembranbündel entstehen zwischen den einzelnen Hohlfasermembranen Zwischenräume, durch die ebenfalls Flüssigkeiten und/oder Gase geleitet werden können. Ein Hohlfasermembranbündel weist vorzugsweise mindestens 50 bis 20.000 Hohlfasermembranen auf. Typische Durchmesser von Hohlfasermembranbündel liegen im Bereich von 15 mm bis 50 mm. Das Hohlfasermembranbündel welches im erfindungsgemäßen Verfahren Anwendung findet, ist nicht vergossen, d.h. die Enden des Hohlfasermembranbündels sind nicht in einem Gießharz vergossen.

Der Zusammenschluss einer Vielzahl der Hohlfasermembranen zu einem Hohlfasermembranbündel ergibt eine Packung von Hohlfasermembranen, in der die Hohlfasermembranen in einer durch die Packung vorgegebenen Dichte aneinander anliegen. Hohlfasermembranbündel bilden in einer derartigen Packung eine Widerstandskraft aus. Dies bedeutet, dass ein Hohlfasermembranbündel, komprimierbar ist und bei Kompression eine Rückstellkraft aufbaut. Komprimierte Hohlfasermembranbündel sind bestrebt in einen entspannten Zustand zurückzukehren. Die Rückstellkraft steht insbesondere auch mit einer Wellenprägung der Hohlfasermembranen im Zusammenhang, die in der Herstellung von Hohlfasermembranen angewendet wird. Entsprechende Verfahren zur Herstellung von Hohlfasermembranen und Hohlfasermembranbündel sind aus dem Stand der Technik beispielsweise aus DE 100 07 327 A1 bekannt.

Weiterhin zeichnen sich Hohlfasermembranbündel, wie sie im erfindungsgemäßen Verfahren zur Anwendung kommen dadurch aus, dass sie ein erstes Ende und ein zweites Ende aufweisen, wobei das erste Ende vom zweiten Ende verschieden ist.

Gemäß des Verfahrens der vorliegenden Erfindung weist das Hohlfasermembranbündel am zweiten Ende verschlossene Hohlfasermembranen auf. Insbesondere sind die Öffnungen der Lumen der Hohlfasermembranen an dem zweiten Ende des Hohlfasermembranbündels endseitig verschlossen, insbesondere so verschlossen, dass keine Flüssigkeit und/oder Gas endseitig aus dem Lumen in die Umgebung austreten oder aus der Umgebung in das Lumen eintreten.

Methoden zum Verschließen der Hohlfasermembranen an einem Ende des Hohlfasermembranbündels sind im Stand der Technik bekannt. Die Hohlfasermembranen können durch Wärmeeinwirkung, z.B. über Wärmestrahlung oder Wärmekontakt, durch Harze, oder über Laserstrahlung verschlossen werden. Vorliegend ist ein Wärmekontaktverfahren an einer Aluminiumfolie bevorzugt. Beispielsweise kann ein Hohlfasermembranbündel an den Enden der Hohlfasermembranen auf einer Heizplatte bei 250°C bis 350°C auf einer Seite geschmolzen werden. Zwischen Heizplatte und den Enden der Hohlfasermembranen wird eine Aluminiumfolie als Trennfolie positioniert, die nach Abkühlen wieder von dem Ende des Hohlfasermembranbündels abgezogen werden kann. Durch den Schmelzvorgang an der Heizplatte werden die Öffnungen der Lumen an den Enden der Hohlfasermembranen zugeschmolzen. Dadurch ergibt sich ein flüssigkeitsdichter Verschluss an dem ersten Enden der Hohlfasermembranen.

In einem weiteren Schritt des erfindungsgemäßen Verfahrens wird ein Gehäuse zur Aufnahme des Hohlfasermembranbündels aufweisend ein erstes Ende und ein zweites Ende, bereitgestellt, wobei das erste Ende mindestens einen Flüssigkeitseinlass aufweist.

Unter dem Begriff "Gehäuse" im Sinne der vorliegenden Anmeldung ist ein Hohlkörper der für die Aufnahme einer Vielzahl von Hohlfasermembranen vorgesehen ist zu verstehen. Wird ein Hohlfasermembranbündel bestehend aus einer Vielzahl von Hohlfasermembranen in ein Gehäuse eingebracht so verbleibt ein Raum im Gehäuse, zwischen den Hohlfasermembranen und zwischen Gehäuseinnenwand und Außenseite der Hohlfasermembranen, der von Flüssigkeiten durchströmt werden kann. Geeignete Gehäuse können eine längliche Ausdehnung aufweisen, so dass eine Erstreckungsachse eines Gehäuses länger ist als eine zweite und dritte Erstreckungsachse und damit als Längsachse des Gehäuses bezeichnet werden kann. Entsprechend der Längsausdehnung eines vorgesehenen Gehäuses kann dieses in bevorzugten Ausrichtungen, vertikal und horizontal verwendet werden.

In einer bevorzugten Form ist ein entsprechendes Gehäuse zylindrisch, z.B. als Hülse ausgestaltet. Entsprechende hülsenförmige Gehäuse können an wenigsten einem der Enden offen sein, so dass ein Hohlfasermembranbündel in die Hülse eingebracht werden kann. Anschließend kann das Gehäuse mit entsprechenden Endkappen oder Anschlussstücken verschlossen oder kombiniert werden. Entsprechende Hülsen, die als Gehäuse für Hohlfasermembranbündel dienen sind aus dem Bau von Dialysatoren im Stand der Technik bekannt. Entsprechende Gehäuse bestehen vorzugsweise aus einem biegesteifen Kunststoffmaterial, wie z.B. Polykarbonat, Polypropylen oder Polyoxymethylen oder aus Metallen, wie z.B. Edelstahl oder Aluminium.

In dem Verfahren der vorliegenden Erfindung weist das Gehäuse an einem Ende des Gehäuses mindestens einen ersten Flüssigkeitseinlass auf. Der Flüssigkeitseinlass ist dafür vorgesehen Flüssigkeit in das Innere des Gehäuses und/ oder in das Innere des Hohlfasermembranbündels beziehungsweise der Hohlfasermembranen einströmen zu lassen. Der mindestens eine Flüssigkeitseinlass kann einen zylindrischen Zugang zu dem Gehäuse an dem einen Ende des Gehäuses darstellen. Der Flüssigkeitszugang kann aber auch ein offenes Ende eines zylindrischen Gehäuses darstellen.

Weiterhin wird gemäß des erfindungsgemäßen Verfahrens zumindest ein Flüssigkeitsauslass bereitgestellt, der von dem mindestens einen Flüssigkeitseinlass beabstandet ist. "*Beabstandet*" meint im Sinne der vorliegenden Anmeldung, dass Flüssigkeitseinlass und Flüssigkeitsauslass so weit von einander entfernt sind, dass durch den Flüssigkeitseinlass einströmende Flüssigkeit einen Teil des Gehäuses und/ oder einen Teil des Hohlfasermembranbündels durchströmt. Insbesondere ist vorgesehen, dass dabei ein Teil des Gehäuses durchströmt wird, der für die Aufnahme eines Hohlfasermembranbündels vorgesehen ist. Der Flüssigkeitsauslass dient insbesondere zum Ableiten von Permeat, insbesondere Ultrafiltrat.

In einem weiteren Schritt des erfindungsgemäßen Verfahrens wird zumindest eine Kompressionsvorrichtung bereitgestellt, die in der Lage sind ist Hohlfasermembranbündel in wenigstens einem Teilbereich des Hohlfasermembranbündels zu komprimieren. Hohlfasermembranbündel zeichnen sich dadurch aus, dass sie deformierbar und insbesondere komprimierbar sind. Beim Komprimieren werden die Hohlfasermembranen im Hohlfasermembranbündel in eine höhere Packungsdichte gebracht. Als Packungsdichte ist dabei die Raumausfüllung von Hohlfasermembranen in einem Hohlfasermembranbündel zu bezeichnen, das in ein Gehäuse eingebracht wurde. Die Packungsdichte von Hohlfasermembranen ist die Summe der Querschnittsflächen der einzelnen Hohlfasermembranen dividiert durch die Gesamtquerschnittsfläche, die alle Hohlfasermembranquerschnittsflächen in einer Anordnung umgrenzt. In der Regel ist dies der Gehäusequerschnitt. Bei Hohlfasermembranen und Gehäusegeometrien mit kreisrunden Querschnitt berechnet sich die Packungsdichte nach folgender Formel:

$$\delta_{(Packungsdichte)} = n \cdot \frac{d^2_{(Faser)}}{d^2_{(Gehäuse)}}$$

$d_{(Faser)}$: ist der mittlere Außendurchmesser der unbelasteten Hohlfasermembran

$d_{(Filter)}$: ist der Innendurchmesser des Gehäuses

$n$: ist die Anzahl der Hohlfasermembranen im Gehäuse

Der Begriff unbelastete Hohlfasermembran bezeichnet eine einzelne freie Hohlfasermembran. Im Gehäuse können unter Kompressionswirkung die Hohlfasermembranen deformiert werden, d.h. unter Belastung einen deformierten Querschnitt annehmen. Insbesondere können sich dadurch Packungsdichten von größer als 100% ergeben. Zur Berechnung der Packungsdichte wird aber stets vom Durchmesser der unbelasteten Hohlfasermembran ausgegangen.

Als "*Kompressionsvorrichtung*" dient ein Mittel dass in der Lage ist die Packungsdichte der Hohlfasermembranen in dem Hohlfasermembranbündel zu erhöhen. Kompressionsvorrichtungen mit denen Hohlfasermembranbündel verdichtet werden können sind im Stand der Technik bekannt. Dies können z.B. elastische Ringe sein, die ein Hohlfaserbündel an mindestens einer Stelle des Hohlfasermembranbündels umschließen und das Hohlfasermembranbündel gemäß einer aufgebrachten Vorspannung des elastischen Materials der Ringe verdichten. Weiterhin können Druckmanschetten oder Druckkissen um das Hohlfasermembranbündel gelegt werden oder in das Hohlfasermembranbündel eingebracht werden, so dass bei Druckbeaufschlagung der Druckmanschette oder des Druckkissens die Packungsdichte der Hohlfasermembranen in dem Hohlfasermembranbündel erhöht wird. Insbesondere kann auch vorgesehen sein, dass das Hohlfasermembranbündel durch besondere geometrische Ausgestaltung des Gehäuses oder Teilen des Gehäuses, insbesondere durch mindestens einen Flüssigkeitseinlass oder durch mindestens einen Flüssigkeitsauslass aus ihrer Lage geschoben und in einen komprimierten Zustand gebracht werden um dadurch zumindest einen Teil des Hohlfasermembranbündels zu komprimieren. Dies kann beispielsweise durch einen Dorn und/ oder eine Hülse geschehen, welche ins Zentrum des Hohlfasermembranbündels und parallel zur Längsachse des Gehäuses eingebracht wird.

Vorzugsweise ist die zumindest eine Kompressionsvorrichtung so ausgestaltet, dass zumindest der Teilbereich des Hohlfasermembranbündels, welche sich an das erste offene Ende anschließt, komprimiert werden kann.

In einem weiteren Schritt des erfindungsgemäßen Verfahrens wird das Hohlfasermembranbündel in das Gehäuse eingebracht, indem das erste Ende des Hohlfasermembranbündels mit den endseitig offenen Lumen der Hohlfasermembranen zu dem zumindest einen ersten Flüssigkeitseinlass am ersten Ende des Gehäuses ausgerichtet ist. Gemäß dieser Anordnung des eingebrachten Hohlfasermembranbündels sind Flüssigkeitseinlass und die offenen Enden der Lumen der Hohlfasermembranen benachbart angeordnet, so dass eine Flüssigkeitsverbindung zwischen einströmender Flüssigkeit und den offenen Lumen der Hohlfasermembranen vorhanden ist und die einströmende Flüssigkeit in das Innere der Hohlfasermembranen durch die offenen Lumen eindringen kann.

Das Einbringen des Hohlfasermembranbündels in das Gehäuse kann dabei mit Hilfe einer reibarmen Zwischenfolie erfolgen in die ein zu untersuchendes Hohlfasermembranbündel eingeschlagen ist. Das in die Folie eingeschlagene Hohlfasermembranbündel weißt dabei einen geringeren Durchmesser auf, als der Gehäuseinnendurchmesser, so dass das eingeschlagene Holfasermembranbündel in das Gehäuse eingeschoben werden kann. Anschließend kann die reibungsarme Folie die zwischen Gehäuseinnenwand und Hohlfasermembranbündel liegt herausgezogen werden, so dass das Hohlfasermembranbündel in dem Gehäuse verbleibt. Bevorzugt ist eine Ausführungsform bei der das Hohlfasermembranbündel teilweise komprimiert in das Gehäuse eingebracht wird. In dem Gehäuse selber richten sich die Hohlfasermembranen entsprechend der Kompressionsspannung des Hohlfasermembranbündels weitgehend raumfüllend aus und entspannen sich. Im Sinne der vorliegenden Anmeldung wird das Einbringen eines Hohlfasermembranbündels in ein Gehäuse unter Kompressionsspannung des Hohlfasermembranbündels, so dass die Hohlfasermembranen den Raum des Gehäuses weitgehend ausfüllen auch als ein "*Einformen*" des Hohlfasermembranbündels in das Gehäuse bezeichnet.

In einem weiteren Schritt des erfindungsgemäßen Verfahrens wird eine Prüfflüssigkeit bereitgestellt. Im Sinne der vorliegenden Anmeldung ist unter dem Begriff "*Prüfflüssigkeit*" eine Flüssigkeit zu verstehen, mit der eine Membran hinsichtlich einer Permeationseigenschaft untersucht werden kann. Solche Prüfflüssigkeiten können z.B. wässrige Lösungen, reines Wasser sein, Blutplasma oder Blut sein. Die Prüfflüssigkeit zeichnet sich dadurch aus, dass sie membrangängig ist, oder dass wenigstens ein Teil der Prüfflüssigkeit membrangängig ist. Unter dem Begriff "membrangängig" ist im Sinne der vorliegenden Anmeldung zu verstehen, dass die Prüfflüssigkeit, bzw. ein Teil der Prüfflüssigkeit vom Lumen der Hohlfasermembran durch die Membranwand hindurch in die äußere Umgebung der Hohlfasermembran permeieren kann.

Die Prüfflüssigkeit wird vorzugsweise in einem Reservoir bereitgestellt und gegebenenfalls temperiert. Im Prinzip kann jede Temperatur gewählt werden, um das erfindungsgemäße Verfahren ausführen zu können. Insbesondere sind Temperaturen von 10°C bis 90°C für die Durchführung des Verfahrens denkbar. Insbesondere sind auch Temperaturen von 20°C bis 40°C für die Durchführung des Verfahrens vorgesehen.

In einem weiteren Schritt des erfindungsgemäßen Verfahrens wird mindestens eine erste Prüfflüssigkeit durch den zumindest einen Flüssigkeitseinlass am ersten Ende des Gehäuses in das Innere des Gehäuses und/ oder des Hohlfasermembranbündels und/ oder des Hohlfasermembranen geleitet. Durch diesen Vorgang wird der Zwischenraum zwischen den Hohlfasermembranen sowie die Lumen der Hohlfasermembranen zumindest teilweise, bevorzugt im Wesentlichen vollständig, durchspült.

In einem weiteren Schritt des erfindungsgemäßen Verfahrens wir das im Gehäuse befindliche Hohlfasermembranbündel in zumindest einem Teilbereich des Hohlfasermembranbündels mittels der zumindest einen Kompressionsvorrichtung komprimiert. Insbesondere wird dabei der Teilbereich des Hohlfasermembranbündels komprimiert, der sich dem ersten Ende des Hohlfasermembranbündels anschließt. Die Kompression verursacht einen Anstieg der Packungsdichte der Hohlfasermembranen in diesem Teilbereich gegenüber dem nicht komprimierten Teilbereich des Hohlfasermembranbündels. Die Kompression bewirkt dadurch auch einen Strömungswiderstand im Zwischenraum zwischen den Hohlfasermembranen. Es ist vorgesehen, die Kompression so einzustellen, dass im Zwischenraum zwischen den Hohlfasermembranen ein Fluss von Flüssigkeit weitgehend unterbunden wird. Der komprimierte Teilbereich des Hohlfasermembranbündels verursacht in diesem Fall, dass einströmende Flüssigkeit am ersten Ende des Gehäuses in das Innere des Gehäuses in die offenen Lumen der Hohlfasermembranen eindringt und das Eindringen der Flüssigkeit in den Zwischenraum zwischen den Hohlfasermembranen erschwert, oder unterbunden wird.

Die Kompression darf dabei nicht so stark sein, dass Hohlfasermembranen beschädigt werden, insbesondere darf der Flüssigkeitsraum in den Lumen der Hohlfasermembranen nicht beeinträchtigt werden. Eine Deformation der Hohlfasermembranen kann aber toleriert werden. Als Maß für die Kompression des Hohlfasermembranbündels dient bei vorgegebener Anzahl und Geometrie der Hohlfasermembranen die Packungsdichte. Es wurde gefunden, dass eine Packungsdichte von mindestens 80% in dem komprimierten Teilbereich des Hohlfasermembranbündels, bei den für das erfindungsgemäße Verfahren vorgesehenen Eingangsdrücken der Prüfflüssigkeit, den Fluss zwischen den Hohlfasermembranen wirksam unterbinden kann. Bei Packungsdichten oberhalb von 150% laufen die Hohlfasermembranen, je nach Membranmaterial, bereits Gefahr beschädigt zu werden. Vorzugsweise liegen die Packungsdichten im komprimierten Teilbereich des Hohlfasermembranbündels im Bereich von größer 80% bis 150%, bevorzugt 85% bis 120%, noch bevorzugter 90% bis 110%. Die Packungsdichte im nicht komprimierten Teilbereich des Hohlfasermembranbündels unterscheidet sich gegenüber dem komprimierten Teilbereich, da in diesem Teilbereich ein Fluss von Flüssigkeit im Raum zwischen den Hohlfasermembranen erforderlich ist. Packungsdichten im nicht komprimierten Teilbereich können im Bereich von mehr als 20% bis weniger als 70% liegen. Oberhalb einer Packungsdichte von 70% ist bei gegebenem notwendigem Druck der einströmenden Prüfflüssigkeit die Strömung zwischen den Hohlfasermembranen schon nachteilig eingeschränkt.

In einem weiteren Schritt des erfindungsgemäßen Verfahrens wird die Permeationseigenschaft, insbesondere die Ultrafiltrationsrate und/ oder der Ultrafiltrationskoeffizient der Hohlfasermembranen bestimmt, indem ein Messwert, insbesondere die Menge an Prüfflüssigkeit gemessen wird, die an zumindest einen Flüssigkeitsauslass, im Besonderen pro Zeiteinheit und bezogen auf die transmembrane Druckdifferenz anfällt.

Der Begriff "*Permeationseigenschaft*" einer Membran bezeichnet im Sinne der vorliegenden Anmeldung die Charakteristik eines transmembranen Stoffübergangs, die mit einer Permeation eines Stoffes durch eine Membranwand im Zusammenhang steht. Eine Permeationseigenschaft einer Membran gibt Auskunft über die Porenstruktur der Membran und wird als Maß verstanden, mit der die Membran hinsichtlich ihrer porösen Struktur charakterisiert werden kann. Eine durch den transmembranen Stoffübergang beobachtete Permeation wird im Allgemeinen in Relation weiterer Größen betrachtet. Insbesondere wird im Sinne der vorliegenden Erfindung unter dem Begriff auch verstanden, wieviel Flüssigkeit durch eine Permeation über die Membran in einem Zeitabschnitt abgetrennt werden kann. Die Menge der permeierten Flüssigkeit pro Zeiteinheit entspricht der Ultrafiltrationrate. Bezieht man die Ultrafiltrationsrate wiederum auf die transmembrane Druckdifferenz, so gibt dieser Permeationswert den Ultrafiltrationskoeffizienten an. Im Sinne der vorliegenden Erfindung werden die Ultrafiltrationsrate und der Ultrafiltrationskoeffizient als eine Permeationseigenschaft verstanden, die eine Hohlfasermembran beschreiben.

Insbesondere wurde in einer Ausführungsform der Erfindung gefunden, dass das erfindungsgemäße Verfahren für die Bestimmung der Ultrafiltrationsrate, insbesondere dem Ultrafiltrationskoeffizienten der untersuchten Hohlfasermembranen verwendet werden kann. Zur Bestimmung der Ultrafiltrationsrate ist es notwendig, dass die einströmende Prüfflüssigkeit, die auf das Ende des Hohlfasermembranbündels mit den endseitig unverschlossenen Hohlfasermembranen trifft, im Wesentlichen nur in das Lumen der Hohlfasermembranen einströmt und nicht in den Zwischenraum zwischen den Hohlfasermembranen. Dies kann erreicht werden, wie vorab beschrieben, indem das Hohlfasermembranbündel, das den Bereich der endseitig unverschlossenen Hohlfasermembranen umfasst komprimiert wird. Der komprimierte Bereich des Hohlfasermembranbündels stellt damit einen "Pseudoverguss" da. Dies bedeutet, dass die Kompression des Hohlfasermembranbündels die Funktionalität eines Harzvergusses des Hohlfasermembranbündels darstellt, indem nur ein Flüssigkeitsstrom im Inneren der Hohlfasermembranen möglich ist. Die Flüssigkeitsströmung zwischen den Fasern im komprimierten Bereich des Hohlfasermembranbündels ist dagegen stark erschwert oder nicht möglich. Je stärker dabei die Kompression ist, desto geringer ist der Fluss im Raum zwischen den Hohlfasermembranen.

Die durch die offenen Enden der Hohlfasermembranen eingetretene Prüfflüssigkeit durchströmt das Innere der Hohlfasermembranen. Da die Enden der Hohlfasermembranen am zweiten Ende des Hohlfasermembranbündels verschlossen sind, permeiert die Prüfflüssigkeit im nicht komprimierten Teil des Hohlfasermembranbündels durch die Membranwand hindurch als sogenanntes Ultrafiltrat in den Raum zwischen den Hohlfasermembranen. Der Raum zwischen den Hohlfasermembranen ist mit dem einen weiteren Flüssigkeitsauslass verbunden, so dass das Ultrafiltrat aus dem Gehäuse abgeleitet werden kann.

In einer weiteren Ausführung gemäß des ersten Aspekts der Erfindung wird eine Permeationseigenschaft der Hohlfasermembran, insbesondere die Ultrafiltrationsrate und/ oder des Ultrafiltrationskoeffizienten durch mehrfaches oder kontinuierliches Messen eines Messwertes, insbesondere der Flüssigkeitsmenge, die an zumindest einen Flüssigkeitsauslass während des Zuströmens der wenigstens einen Prüfflüssigkeit in das Gehäuse, anfällt, zeitabhängig aufgenommen, um einen zeitabhängigen Messwerteverlauf zu erhalten. Die Vielzahl der zeitabhängig aufgenommenen Messwerte, oder der kontinuierliche Messwerteverlauf eignet sich dazu Messfehlerschwankungen auszugleichen. Werden sonstige Parameter der Verfahrensdurchführung konstant gehalten, z.B. Temperatur oder transmembrane Druckdifferenz, so ergibt sich für die Aufnahme der Ultrafiltratmenge in Abhängigkeit von der Zeit eine lineare Korrelation. Entsprechend kann an den Messwerteverlauf eine lineare Regressionsgerade angenähert werden und aus der Steigung die Ultrafiltrationsrate bestimmt werden. Normiert auf die voreingestellt konstante transmembrane Druckdifferenz ist der Ultrafiltrationskoeffizient ableitbar.

In einer weiteren Ausführung gemäß des ersten Aspekts der Erfindung erstreckt sich der komprimierte Teil des Hohlfasermembranbündels in axialer Richtung über einen Längenabschnitt von 10 mm bis 150 mm gemessen ab dem ersten Ende des Hohlfasermembranbündels. Unterhalb einer Länge von 10 mm liefert die Kompression des Hohlfasermembranbündels abhängig von der Packungsdichte der Hohlfasermembranen keine ausreichende Wirkung den Fluss der Prüfflüssigkeit im Raum zwischen den Hohlfasermembranen zu unterbinden. Oberhalb einer Länge von 150 mm wird die Aussagekraft der Messergebnisse als Qualitätsmerkmal für den Bau von Hämodialysatoren verschlechtert, da die Hohlfasermembranbündel in der Produktion von Dialysefiltern selbst nur einer Länge von ca. 250 mm bis 300 mm haben. Ist der Bereich an dem die Ultrafiltration der Hohlfasermembran stattfindet zu gering, ist mit einem unerwünschten Fehlerbereich der Messmethode zu rechnen.

In einer weiteren Ausführung gemäß des ersten Aspekts der Erfindung weist der zumindest eine Flüssigkeitsauslass eine erste und eine zweite Öffnung auf und der Flüssigkeitsauslass ist so am Gehäuse angebracht, dass eine Flüssigkeitsverbindung zwischen der ersten Öffnung, die im nicht komprimierten Teil des Hohlfasermembranbündels liegt und der zweiten Öffnung, die außerhalb des Hohlfasermembranbündels liegt, entsteht, wobei sich der Flüssigkeitsauslass insbesondere durch den komprimierten Teilbereich des Hohlfasermembranbündels hindurch erstreckt.

Insbesondere ist der zumindest eine Flüssigkeitsauslass in einer Ausführungsform am Gehäuse so konstruiert, dass er das Hohlfasermembranbündel an dem ersten Ende mit den endseitig offenen Hohlfasermembranen, in axialer Ausrichtung der Hohlfasermembranen, durchdringt, und das Hohlfasermembranbündel in diesem Teil des Hohlfasermembranbündels komprimiert. Für diese Ausführung ist vorgesehen, dass der Flüssigkeitsauslass die Form einer Hülse annimmt. Gleichzeitig dient in dieser Ausführung der Flüssigkeitsauslass als Kompressionsvorrichtung für das Hohlfasermembranbündel, indem er die Hohlfasermembranen in dem entsprechenden Abschnitt des Hohlfasermembranbündels verdrängt und somit eine höhere Packungsdichte bewirkt. In dieser Ausführung stellt der Flüssigkeitsauslass demzufolge eine Flüssigkeitsverbindung mit dem Raum der zwischen den Hohlfasermembranen im nicht komprimierten Teil des Hohlfaserbündels her, um dort anfallendes Ultrafiltrat abzuleiten.

Um die Funktion von Flüssigkeitsauslass und Kompression gleichzeitig zu bewirken ist in einer weiteren Ausführungsform gemäß dem ersten Aspekt der Erfindung vorgesehen, den Flüssigkeitsauslass beweglich am Gehäuse aufzunehmen. In einem weiteren Schritt des erfindungsgemäßen Verfahrens wird der Flüssigkeitsauslass in axialer Richtung zu der Längsachse des Gehäuses bewegt und in einen Abschnitt des Hohlfasermembranbündels geschoben, so dass die Hohlfasermembranen aus ihrer Lage verdrängt und die Packungsdichte erhöht wird. Die Einführung des Flüssigkeitsauslasses in das Hohlfasermembranbündel kann mittels eines Hilfsmittels vorgenommen werden. Das Hilfsmittel kann in dem Hohlfasermembranbündel verbleiben, oder nach Einführung des Flüssigkeitsauslasses wieder entfernt werden

Gemäß dieser Ausführungsform ist vorgesehen, den Flüssigkeitsauslass dornförmig auszubilden oder den Flüssigkeitsauslass als Hülse auszubilden, der mit Hilfe eines Dorns in das Hohlfasermembranbündel geschoben wird. Eine dornförmige Geometrie bewirkt, dass die Hohlfasermembranen beschädigungsfrei verschoben werden können um die Packungsdichte im Kompressionsbereich zu erhöhen.

Gemäß einer weiteren Ausführung ist vorgesehen die Kompression des Hohlfasermembranbündels durch eine Kombination verschiedener Kompressionsvorrichtungen zu erzeugen. Entsprechend kann vorgesehen sein die Kompression z.T. durch ein in das Hohlfasermembranbündel eingeschobene Hülse aus Flüssigkeitsauslass und elastischen Ringen oder einer Druckmanschette zu erzeugen.

Es ist vorgesehen, dass die Packungsdichte im nicht komprimierten Teil des Hohlfaserbündels weniger als 70% beträgt. Im Rahmen der verwendeten Terminologie der vorliegenden Anmeldung bezeichnen die Begriffe "komprimiert" und "nicht komprimiert" Abschnitte des Hohlfasermembranbündels, die relativ zueinander in unterschiedlich hoher Packungsdichten vorliegen. Die Begriffe sind nicht zwangsläufig als absolute Begriffe zu verstehen. Demnach kann ein Abschnitt des Hohlfaserbündels, das eine Packungsdichte von 60% aufweist als nicht komprimiert verstanden werden, wenn ein zweiter Abschnitt des Hohlfasermembranbündels eine Packungsdichte von 80% aufweist.

In einer weiteren Ausführung gemäß des ersten Aspekts der Erfindung ist vorgesehen, dass am Gehäuse wenigstens eine Öffnung zur Entlüftung angebracht ist. Für die Reproduzierbarkeit der Ultrafiltrationsmessung kann eine vollständige Entlüftung des Innenraumes des Gehäuses notwendig sein. Entsprechend ist in einem Schritt des Verfahrens, gemäß dem ersten Aspekt der Erfindung vorgesehen, während dem Zuströmen der Prüfflüssigkeit in das Gehäuse verdrängte Luft über den Gasauslass abzuleiten. Je nach Porenbeschaffenheit der untersuchten Hohlfasermembranen kann die Ableitung von verdrängter Luft notwendig sein, sofern sie nicht vollständig über den Flüssigkeitsauslass aus dem Gehäuse ausgeleitet werden kann. Das Gehäuse kann die Entlüftungsöffnung z.B. als Lochkranz am Gehäuse aufweisen. Die Entlüftungsöffnung ist in unmittelbarer Nähe zum dem Ende des Hohlfasermembranbündels angeordnet, dessen Hohlfasermembranen endseitig verschlossen sind. Die Entlüftungsöffnung kann nach dem Konditionieren, d.h. nachdem das Gehäuse entlüftet wurde, wieder verschlossen werden.

In einer weiteren Ausführung, gemäß dem ersten Aspekt der Erfindung ist wenigstens ein weitere Öffnung im Gehäuse gegenüberliegend zum nicht komprimierten Abschnitt des eingeformten Hohlfasermembranbündels vorgesehen, die in unmittelbarer Angrenzung zum komprimierten Abschnitt des Hohlfasermembranbündels liegt. Mit Hilfe der Öffnungen kann ein Strom von Hilfsluft während der Ultrafiltrationsmessung in das Gehäuse eingeleitet werden, um Ultrafiltrat, das sich umfänglich am Hohlfasermembranbündel sammelt in Richtung des offenen Endes des Flüssigkeitsauslasses abzuleiten.

In einer weiteren Ausführung gemäß des ersten Aspekts der Erfindung ist zumindest ein weiterer Flüssigkeitsauslass am Gehäuse, gegenüberliegend zum komprimierten Abschnitt des eingeformten Hohlfasermembranmoduls vorgesehen, der in unmittelbarer Angrenzung zum nicht komprimierten Abschnitt des Hohlfasermembranbündels liegt. Der Flüssigkeitsauslass kann hilfreich sein um während es Konditioniervorgangs, d.h. während des Durchspülens des Gehäuses mit Prüfflüssigkeit vor Messbeginn, auch im komprimierten Teil eine vollständige Durchspülung im komprimierten Abschnitt des Hohlfasermembranbündels zu erreichen. Zudem ist über diesen Flüssigkeitsauslass eine Abführung von Leckageflüssigkeit während der Messung möglich. Der Begriff Leckageflüssigkeit bezeichnet im Sinne der vorliegenden Anmeldung dabei Prüfflüssigkeit die sich trotz der Kompression des Hohlfasermembranbündels im Zwischenraum zwischen den Hohlfasermembranen eindringt. Es ist unerwünscht, dass Leckageflüssigkeit bis in den nicht komprimierten Teil des Hohlfasermebranbündels vordringen kann, da sie dort die gemessene Menge an Ultrafiltrat verfälschen kann. Der weitere Flüssigkeitsauslass stellt eine Verbindung zum Umgebungsdruck her. Daher fällt der Druck im Zwischenraum zwischen den Hohlfasermembranen im komprimierten Teilbereich des Hohlfasermembranbündels auf das Niveau des Umgebungsdrucks ab. Der nicht komprimierte Teilbereich steht ebenfalls über zumindest einen Flüssigkeitsauslass mit dem Umgebungsdruck in Beziehung. Demzufolge kann sich eventuell auftretende Leckageflüssigkeit im Raum zwischen den Hohlfasermembranen im komprimierten Teilbereich des Hohlfasermembranbündels nicht über den weiteren Flüssigkeitsauslass hinaus in den nicht komprimierten Teilbereich des Hohlfasermembranbündels fortbewegen, weil ein Ausgleich der Druckniveaus durch den weiteren Flüssigkeitsauslass hergestellt wird.

In einer weiteren Ausführungsform des erfindungsgemäßen Verfahrens ist vorgesehen, dass die Kompression des Hohlfasermembranbündes in einem Teilbereich durch mindestens zwei Kompressionsvorrichtungen bewirkt wird. In einer weiteren Ausführungsform können eine oder mehrere, insbesondere zwei Kompressionsvorrichtungen im Inneren des Hohlfasermembranbündels angebracht werden. In einer weiteren Ausführung können eine oder mehrere Kompressionsvorrichtungen, welche das Hohlfasermembranbündel zumindest teilweise umschließen und eine oder mehrere Kompressionsvorrichtungen, welche sich im Inneren des Hohlfasermembranbündels befinden, vorgesehen sein.

Die Kompression durch zwei Kompressionsvorrichtungen hat sich insbesondere bei Messverfahren an Hohlfasermembranen mit einer großen mittleren Porengröße und hohem Ultrafiltrationskoeffizienten als vorteilhaft erwiesen. Das Auftreten von Leckageflüssigkeit im komprimierten Teilbereich des Hohlfasermembranbündels kann insbesondere bei solchen Hohlfasermembranen auch durch Ultrafiltration im komprimierten Teilbereich entstehen. Eine zweite Kompressionsvorrichtung bewirkt eine verbesserte Barriere gegen das Eindringen von Leckageflüssigkeit in den nicht komprimierten Teilbereich des Hohlfasermembranbündels.

In einer weiteren Ausführungsform des erfindungsgemäßen Verfahrens ist vorgesehen, dass der zumindest eine weitere Flüssigkeitsauslass am Gehäuse zwischen den zumindest zwei Kompressionsvorrichtungen angebracht ist. Dadurch wird Leckageflüssigkeit die aus Richtung der einströmenden Flüssigkeit am Flüssigkeitseinlass des Gehäuses nach der ersten Kompressionsvorrichtung auftritt wirksam abgeführt. Zudem wird das Druckniveau im Raum zwischen den Hohlfasermembranen nach der ersten Kompressionsvorrichtung auf Umgebungsdruckniveau angeglichen werden. Die zweite Kompressionsvorrichtung verhindert zusätzlich, dass sich weitere Leckageflüssigkeit im komprimierten Teilbereich in den nicht komprimierten Teilbereich des Hohlfasermembranbündels fortbewegen kann.

In einer weiteren Ausführungsform des ersten Aspekts der Erfindung ist vorgesehen, den Druck der zuströmenden Flüssigkeit auf 50 mbar bis 500 mbar, alternativ 100 bis 300 mbar, alternativ 150 bis 250 mbar einzustellen. Es hat sich gezeigt, dass die vorab geschilderte Kompression des Hohlfasermembranbündes ausreichend hoch ist, um bei den genannten Drücken der einströmenden Prüfflüssigkeit eine ausreichende Sperrwirkung des Flusses im Raum zwischen den Hohlfasermembranen zu bewirken. Die Drücke können damit ausreichend hoch gewählt werden, um für die Bestimmung der Ultrafiltrationsrate ein ausreichend hohes transmembranes Druckgefälle zu erzeugen. Die Ultrafiltrationsrate kann anschließend aus der Menge, bzw. dem Volumen des Ultrafiltrats, das pro Zeiteinheit am Flüssigkeitsauslass gewonnen wird, bestimmt werden. Das Volumen des Ultrafiltrats kann nach gängigen Methoden volumetrisch oder gravimetrisch bezogen auf die Dichte der Prüfflüssigkeit bestimmt werden.

In bestimmten Ausführungen, z.B. je nach Anzahl der Hohlfasermembranen im Gehäuse, dem Hohlfasermembrandurchmesser oder der Porenbeschaffenheit der Hohlfasermembranen kann es sein, dass nach dem vorliegenden Verfahren gemäß einer Ausführungsform nach dem ersten Aspekt der Erfindung Werte für die Ultrafiltrationsrate ermittelt werden, die von standardisierten Verfahren, z.B. der DIN/ EN/ ISO 8637:2014, abweichen. In diesen Fällen können die erhaltenen Messwerte mittels zuvor bestimmter Kalibrierwerte oder mittels einer Kalibrierfunktion korrigiert werden.

Zur Bestimmung von Kalibrierwerten beziehungsweise einer Kalibrierfunktion werden unterschiedliche Hohlfasermembran-Filtermodule mit Hohlfasermembranen unterschiedlicher Porenbeschaffenheit hinsichtlich des Ultrafiltrationskoeffizienten nach DIN/ EN/ ISO 8634 untersucht. Anschließend werden entsprechende Hohlfasermembranbündel mit Hohlfasermembranen, die den Hohlfasermembranen aus den jeweiligen Hohlfasermembran-Filtermodulen identisch sind, nach dem erfindungsgemäßen Verfahren bestimmt. Es wurde gefunden, das die Ergebnisse aus dem erfindungsgemäßen Verfahren mit den Werten die an Hohlfasermembran-Filtermodulen nach den Methoden aus dem Stand der Technik gewonnen wurden, korreliert werden können und eine Kalibrierkurve erstellt werden kann. Für den Fall dass die Messungen nach der erfindungsgemäßen Methode eine systematische Abweichung zu anderen Messmethoden insbesondere der Methode nach DIN/ EN / ISO 8637 führt kann entsprechend nach dem vorliegenden erfindungsgemäßen Verfahren eine Anpassung der Messwerte durch eine Korrekturfunktion vorgenommen werden.

In einem zweiten Aspekt betrifft die Erfindung eine Vorrichtung zum Durchführen eines Verfahrens nach einem der Ausführungen nach dem ersten Aspekt der Erfindung. Bei der Vorrichtung handelt es sich um eine Messvorrichtung mit der die eine Permeationseigenschaft, insbesondere die Ultrafiltrationsrate und/oder der Ultrafiltrationskoeffizient von Hohlfasermembranen an einem Hohlfasermembranbündel bestimmt werden kann. Die Vorrichtung weist auf:

(a) mindestens ein Reservoir zur Aufnahme einer Prüfflüssigkeit,

(b) ein Gehäuse mit einem ersten Ende und einem zweiten Ende zur Aufnahme eines Hohlfasermembranbündels, wobei das erste Ende des Gehäuses zumindest einen Flüssigkeitseinlass aufweist,

(c) zumindest eine Verbindungsvorrichtung, insbesondere Pumpmittel, zum Zuführen der zumindest einen Prüfflüssigkeit von dem zumindest einem Reservoir ins Innere des Gehäuses und/oder des Hohlfasermembranbündels, und/ oder der Hohlfasermembranen.

(d) Zumindest eine Kompressionsvorrichtung zum Komprimieren von zumindest einem Teilbereich des Hohlfasermembranbündels, wobei es sich bei der zumindest einen Kompressionsvorrichtung um einen Dorn und/ oder eine Hülse handelt, welche ins Zentrum des Hohlfasermembranbündels und parallel zur Längsachse des Gehäuses eingebracht werden kann,

(e) zumindest einen Flüssigkeitsauslass

(f) Mittel zur zeitabhängigen Messung der Ultrafiltrationsrate und/ oder des Ultrafiltrationskoeffizienten an zumindest einem Flüssigkeitsauslass, wobei die Ultrafiltrationsrate die Menge der permeierten Flüssigkeit pro Zeiteinheit ist und der Ultrafiltrationskoeffizient der Ultrafiltrationsrate bezogen auf die transmembrane Druckdifferenz entspricht.

Die erfindungsgemäße Vorrichtung bietet den Vorteil, dass Hohlfasermembranbündel hinsichtlich ihrer Permeationseigenschaft, insbesondere hinsichtlich der Ultrafiltrationsrate und/ oder des Ultrafiltrationskoeffizienten untersucht werden können, ohne dass die Hohlfasermembranmodule in Testfiltermodule vergossen werden müssen und zu Filtermodulen verbaut werden müssen. Mit der erfindungsgemäßen Vorrichtung lassen sich Hohlfasermembranbündel innerhalb weniger Minuten hinsichtlich einer ihrer Permeationseigenschaft, insbesondere hinsichtlich der Ultrafiltrationsrate und des Ultrafiltrationskoeffiezenten vermessen.

Die Vorrichtung eignet sich daher insbesondere, um in der kontinuierlichen Produktion von Hohlfasermembranen die Produktspezifikationen der hergestellten Hohlfasermembranen hinsichtlich einer der vorbestimmten Permeabilitätseigenschaften zu überwachen.

In einer weiteren Ausführungsform der erfindungsgemäßen Vorrichtung weist die Vorrichtung weiterhin Mittel zur Bestimmung des Drucks an zumindest einem Flüssigkeitseinlass auf. Entsprechende Mittel sind im Stand der Technik bekannt und können z.B. einen Drucksensor darstellen. Mit Hilfe des Drucksensors kann der Eingangsdruck der einströmenden Prüfflüssigkeit an dem zumindest einen Flüssigkeitseinlass bestimmt werden. Zusammen mit einer Pumpe, die die Prüfflüssigkeit zum dem wenigstens einen Flüssigkeitseinlass fördert kann ein vorbestimmter Druck der zuströmenden Prüflflüssigkeit eingestellt werden. Optional kann ein weiteres Mittel zur Bestimmung des Drucks, insbesondere ein Drucksensor, an dem zumindest einem weiteren Flüssigkeitsauslass vorgesehen sein, um die genaue transmembrane Druckdifferenz zu ermitteln. Im Allgemeinen entspricht der Druck an einem Flüssigkeitsauslass dem Umgebungsdruck, so dass für die Ermittlung der transmembranen Druckdifferenz der aufgenommene Druckwert an dem zumindest einen Flüssigkeitseinlass ausreichend ist. Die Ermittlung der transmembranen Druckdifferenz ist für die Bestimmung des Ultrafiltrationskoeffizienten aus der gemessenen Ultrafiltrationsrate notwendig.

Vorteilhafterweise weist in einer weiteren Ausführungsform der erfindungsgemäßen Vorrichtung das Gehäuse in einem endseitigen Teil, der zur Aufnahme des Abschnitts des Hohlfasermembranbündels mit den endverschlossenen Hohlfasermembranen vorgesehen ist, wenigstens einen Gasauslass auf. Der Gasauslass kann als Entlüftungsöffnung dienen durch den während des Messvorgangs abgeschiedene Luft abgeleitet werden kann.

In einer weiteren Ausführungsform der erfindungsgemäßen Vorrichtung weist das Gehäuse in einem Abschnitt, der zur Aufnahme eines nicht komprimierten Teils des Hohlfasermembranbündels vorgesehen ist, wenigstens eine Öffnung als Gaseinlass auf.

Der Gaseinlass kann dazu dienen gegebenenfalls Luft in das Innere des Gehäuses einzuleiten. Mit Hilfe der eingeleiteten Luft kann ein Luftstrom in dem Gehäuse gebildet werden, um anfallendes Permeat, insbesondere Ultrafiltrat im Zwischenraum zwischen den Hohlfasermembranen zum Flüssigkeitsauslass abzuleiten. Die Messung wird umso präziser, je besser die Wasserströme auf den jeweiligen Membranseiten voneinander getrennt werden können. Demzufolge ist es wichtig, dass möglichst das ganze Ultrafiltrat nach innen zum zentralen Flüssigkeitsauslass geleitet wird.

Insbesondere betrifft die Erfindung in einem dritten Aspekt die Verwendung einer erfindungsgemäßen Vorrichtung nach dem zweiten Aspekt der Erfindung zur Anwendung eines erfindungsgemäßen Verfahrens nach dem ersten Aspekt der Erfindung.

In einem vierten Aspekt betrifft die Erfindung die Herstellung von Hohlfasermembran-Filtermodulen, wobei das erfindungsgemäße Verfahren gemäß einer Ausführung des ersten Aspekt der Erfindung dazu dient, um Hohlfasermembran mit einer vorbestimmten Permeationseigenschaft herzustellen.

Das erfindungsgemäße Herstellungsverfahren von Hohlfasermembran-Filtermodulen weist die folgenden Schritte auf:

(a) Festlegen von einem oder mehreren Wertebereichen für wenigstens eine Permeationseigenschaft, insbesondere die Ultrafiltrationsrate und/ oder den Ultrafiltrationskoeffizient, von Hohlfasermembranen, die für die Herstellung von Hohlfasermembran-Filtermodulen vorgesehen sind,

(b) Auswählen von einem oder mehreren Herstellparametern um Hohlfasermembranen mit der wenigstens einen Permeationseigenschaft, oder den mehreren Permeationseigenschaften, mit dem im Schritt (a) festgelegten Wertebereich herzustellen,

(c) Herstellen von Hohlfasermembranen durch ein Spinnverfahren nach einem oder mehreren im Schritt (b) ausgewählten Herstellparametern,

(d) Bündeln der erhaltenen Hohlfasermembranen zu Hohlfasermembranbündeln,

(e) Durchführen eines Verfahrens gemäß einer Ausführung des ersten Aspekts der Erfindung zur Bestimmung der Ultrafiltrationsrate und/ oder des Ultrafiltrationskoeffizienten der Hohlfasermembranen wobei die Ultrafiltrationsrate die Menge der permeierten Flüssigkeit pro Zeiteinheit ist und der Ultrafiltrationskoeffizient der Ultrafiltrationsrate bezogen auf die transmembrane Druckdifferenz entspricht,

(f) Verwendung der Hohlfasermembranbündel für den Bau von Filtermodulen, wenn festgestellt wird, dass die einen oder mehreren Permeationseigenschaften im in Schritt (a) festgelegten Wertebereich liegen.

Da mit dem erfindungsgemäßen Verfahren nach dem ersten Aspekt der Erfindung mit geringem Zeitaufwand eine Permeationsseigenschaft, insbesondere die Ultrafiltrationsrate und/ oder der Ultrafiltrationskoeffizient der hergestellten Hohlfasermembranen bestimmt werden kann, kann auch der Herstellprozess von Hohlfasermembran-Filtermodulen zeitnah gesteuert werden. Insbesondere kann geprüft werden, ob die hergestellten Hohlfasermembranen der vorgegebenen Produktspezifikation entsprechen, so dass die Hohlfasermembranen für den Bau von Hohlfasermembran-Filtermodulen freigegeben werden können.

Für den Fall, dass festgestellt wird, dass die Produktspezifikationen der hergestellten Hohlfasermembranen durch den Herstellprozess nicht eingehalten oder nur knapp eingehalten werden, können entsprechende Rückschlüsse bezüglich der Herstellparameter getroffen werden. Die Herstellparameter können in Folge so angepasst werden, dass die erhaltenen Hohlfasermembranen den Produktspezifikationen wieder entsprechen. Der so regulierte Herstellprozess durchläuft dann wieder die vorgesehenen Schritte (a) bis (f), indem für die hergestellten Hohlfasermembranen entschieden werden kann, diese für den Bau der Hohlfasermembran-Filtermodule zu verwenden.

Das erfindungsgemäße Herstellverfahren eignet sich insbesondere für die Herstellung von Hohlfasermembran-Filtermodulen, die auf Basis der Membranmaterialien Polysulfon (PSU) und Polyvinylpyrrolidon hergestellt werden. Die Spinnlösung, die für die Herstellung solcher Hohlfasermembranen verwendet werden, enthalten Polysulfon (PSU) und Polyvinylpyrrolidon (PVP), gelöst in einem polaren aprotischen Lösungsmittel. Geeignete Lösungsmittel stellen z.B. N-Methyl-pyrrolidon, Dimethylsulfoxid oder DiMethyl-Acetamid da. Es hat sich herausgestellt, dass es bei Ansätzen von Polysulfon /Polyvinylpyrrolidon Spinnlösungen besonders schwierig ist die Permeationseigenschaften, insbesondere die Ultrafiltrationsrate und/ oder den Ultrafiltrationskoeffizienten, von Hohlfasermembranen exakt einzustellen, da diese sehr empfindlich auf Schwankungen der Produktionsparameter reagieren. Das vorliegende erfindungsgemäße Herstellverfahren hat sich für die Herstellung von PSU/ PVP Hohlfasermembranen als besonders vorteilhaft herausgestellt, da es die Möglichkeit bietet, Abweichungen, die durch die Veränderung der Produktionsparameter hervorgerufen werden, in kürzester Zeit zu detektieren und den Herstellprozess entsprechend anzupassen.

## Beschreibung der Erfindung anhand der Figuren

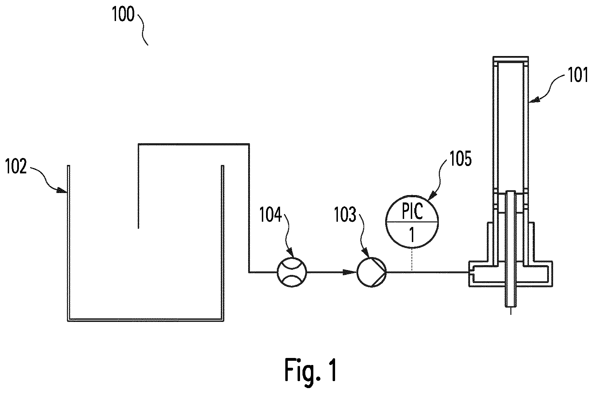

Fig. 1 ist eine schematische Darstellung einer erfindungsgemäßen zur Bestimmung der Ultrafiltrationsrate und/ oder des Ultrafiltrationskoeffizienten von Hohlfasermembranen eines Hohlfasermembranbündels. Die Messvorrichtung (100) weist eine Messkammer (101), ein Wasserbad (102), in dem destilliertes auf 37°C temperiertes Wasser als Prüfflüssigkeit vorgelegt ist, eine Pumpe (103) mit der das Wasser in die Messkammer (101) gefördert wird, einen Durchflussmesser (104), und einen Drucksensor (105) auf. Die Pumpe wird so geregelt, dass das in die Vorrichtung (100) einströmende Wasser einen konstanten Eingangsdruck von 200 mbar aufweist. Die Menge des zugeführten Wassers wird stets mit einem Durchflussmesser (104) ermittelt.

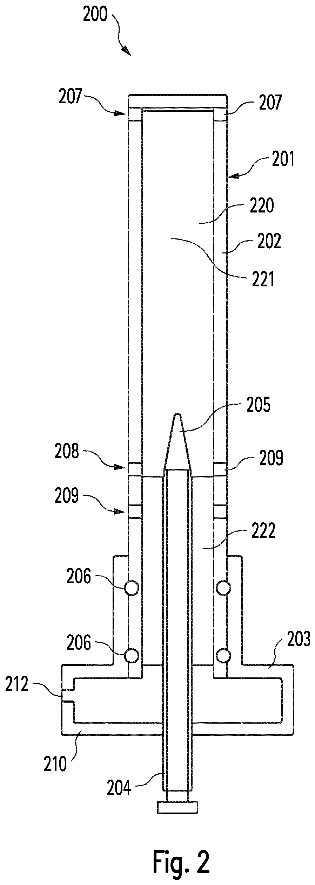

Fig. 2 ist eine detailliertere schematische Darstellung der erfindungsgemäßen Messvorrichtung (200) mit den folgenden Bezeichnungen:

- Gehäuse (201)
- Gehäusehülse (202)
- Aufnahmeteil (203)
- Flüssigkeitsauslass (204)
- Dorn (205)

- Dichtung (206)
- Gasauslass (207)
- Gaseinlass, ausgestaltet als Lochkranz (208)
- weiterer Flüssigkeitsauslass (209)
- Boden des Aufnahmeteils (210)
- Hohlfasermembranbündel (220)
- nicht komprimierter Abschnitt des Hohlfasermembranbündels (221)
- komprimierten Abschnitt des Hohlfasermembranbündels (222)
- Flüssigkeitseinlass (212)

In die Gehäusehülse (202) der Messkammer ist ein Hohlfasermembranbündel eingeformt. Das Gehäuse besteht in dieser Ausführung aus einer Hülse (202), an dem sich ein Aufnahmeteil (203) anschließt, sowie einem hülsenförmigen Flüssigkeitsauslass (204), in den ein Dorn (205) eingeschoben ist. Das Aufnahmeteil (203) weist einen Flüssigkeitseinlass (212) auf.

Die Gehäusehülse (202) ist an einem Ende in das Aufnahmeteil (203) eingeschoben und ist mit 2 O-Ringen (206) abgedichtet. Zusätzlich ist das Gehäuse mit 3 Lochkränzen versehen. Die Lochkränze bilden Gasauslass (207), Gaseinlass (208) und den weiteren Flüssigkeitsauslass (209), um Leckagewasser abzuführen. Als Prüfflüssigkeit wird Wasser verwendet.

Am Boden (210) des Aufnahmeteils (203) ist ein verschiebbarer Dorn (205) mit der den Dorn umschließenden Hülse ausgestaltet als Flüssigkeitsauslass (204) angebracht, der in das Faserbündel (220) hineinreicht. Der Dorn (205) kann wieder entnommen werden. Dabei verbleibt der hülsenförmige Flüssigkeitsauslass (204) im Hohlfasermembranbündel (220).

Das Hohlfasermembranbündel weist im oberen Bereich, in dem sich kein Dorn (203) und/ oder Flüssigkeitsauslass (204) befindet einen nicht komprimierten Abschnitt (221) und im unteren Bereich einen komprimierten Abschnitt (222) auf.

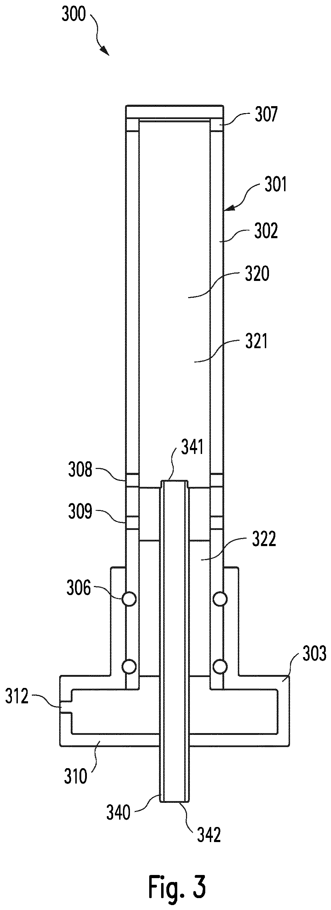

Fig.3 ist eine weitere schematische Darstellung einer erfindungsgemäßen Messvorrichtung (300), die im Wesentlichen mit der Messkammer aus Fig. 2 identisch ist. Für Fig.3 gelten die folgenden Bezeichnungen:

- Gehäuse (301)
- Gehäusehülse (302)
- Aufnahmeteil (303)
- Flüssigkeitsauslass (340)
- erstes Ende des Flüssigkeitsauslasses (341)
- zweites Ende des Flüssigkeitsauslasses (342)
- Dichtungen (306)
- Gasauslass (307)
- Gaseinlass (308)
- weiterer Flüssigkeitsauslass (309)
- Boden des Aufnahmeteils (310)
- Hohlfasermembranbündel (320)
- nicht komprimierter Abschnitt des Hohlfasermembranbündels (321)
- komprimierten Abschnitt des Hohlfasermembran-

- bündels (322)
- Flüssigkeitseinlass (312)

Fig. 3 zeigt eine Ausführung mit einem Flüssigkeitsauslass (340), welcher sich im Hohlfasermembranbündel befindet. Ein erstes Ende (341) des Flüssigkeitsauslasses liegt im Abschnitt des Hohlfasermembranbündels, das nicht komprimiert (321) ist. Das zweite Ende (341) des Flüssigkeitsauslasses liegt außerhalb der Messvorrichtung (300), so dass eine Flüssigkeitsverbindung mit dem Raum zwischen den Hohlfasermembranen im nicht komprimierten Abschnitt (321) des Hohlfasermembranbündels (320) und der Außenseite der Messvorrichtung (300) hergestellt wird. Das aus dem unteren Lochkranz (309) der Hülse auslaufende Leckagewasser wird zurück in das Wasserbecken geführt. Die Hilfsluft kann mittels einer Manschette an dem Gaseinlass (308) zugeführt werden. Der Gasauslass (307) kann je nach Wahl geöffnet oder geschlossen werden. Das Ultrafiltrat wird mittels einer Analysenwaage gewogen und über die Dichte in Milliliter pro Zeiteinheit umgerechnet.

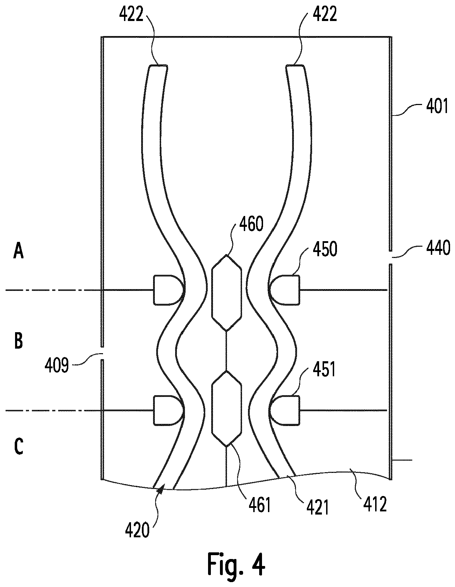

Fig. 4 zeigt eine schematische Darstellung einer weiteren Ausführungsform der erfindungsgemäßen Vorrichtung, mit einer alternativen Kompressionsvorrichtungen mit der der Fluss von Prüfflüssigkeit im Raum zwischen den Hohlfasermembranen unterbunden werden kann. Für Fig. 4 gelten folgende Bezeichnungen:

- Gehäuse (401)
- Flüssigkeitsauslass (440)
- weiterer Flüssigkeitsauslass (409)
- Flüssigkeitseinlass (412)
- Hohlfasermembranen (420, 421)
- Flüssigkeitsdicht verschlossenes Ende der Hohlfasermembranen (422)
- erste Kompressionsvorrichtung (450)
- zweite Kompressionsvorrichtung (451)
- dritte Kompressionsvorrichtung (460)
- vierte Kompressionsvorrichtung (461)

Fig. 4 zeigt zwei Hohlfasermembranen (420, 421), die schematisch für eine Vielzahl von Hohlfasermembranen eines Hohlfasermembranbündels stehen und die an einem Ende (422) flüssigkeitsdicht verschlossen sind. Fig. 4 zeigt weiterhin eine erste Kompressionsvorrichtung (450) und eine zweite Kompressionsvorrichtung (451), welche die Hohlfasermembran umschließt. Weiterhin ist eine dritte Kompressionsvorrichtung (460) innerhalb des Hohlfasermembranbündels, welches durch die Hohlfasermembranen (420, 421) symbolisiert wird, angebracht, Eine vierte Kompressionsvorrichtung (461) befindet sich ebenfalls innerhalb des Hohlfasermembranbündel. In einer Ausführungsform kann vorgesehen sein, dass eine oder mehrere Kompressionsvorrichtungen (450, 451), das Hohlfasermembranbündel umschließend angebracht sind. Die wellenförmige Zeichnung der Hohlfasermembranen (420, 421) gibt schematisch die Kompression des Hohlfasermembranbündels wieder, die durch die Kompressionsvorrichtungen (450, 451, 460, 461) hervorgerufen wird. Die erste und die dritte Kompressionsvorrichtung (450, 460), sowie die zweite und die vierte Kompressionsvorrichtung (451, 461) sind im Wesentlichen auf der gleiche Höhe am beziehungsweise im Hohlfasermembranbündel angebracht, das heißt, sie liegen sich gegenüber.

Die vier angeordneten Kompressionsvorrichtungen teilen das Hohlfasermembranbündel in drei Bereiche ein (A, B, C). Der Bereich C steht dabei mit dem Flüssigkeitseinlass (412) in Verbindung. Der Bereich A steht mit einem Flüssigkeitsauslass (440) in Verbindung. Durch die einströmende Prüfflüssigkeit herrscht im Lumen der Hohlfasermembranen der Druck aus dem Bereich C. Da der Druck im Bereich C durch die zuströmende Flüssigkeit höher ist, als im Zwischenraum zwischen den Hohlfasermembranen im Bereich A, findet eine Ultrafiltration von Prüfflüssigkeit durch die Membranwand hindurch von der Lumenseite der Hohlfasermembranen in den Zwischenraum der Hohlfasermembranen im Bereich C statt. Durch Kompression des Hohlfasermembranbündels mittels der Kompressionsvorrichtung (451) und/ oder (461) wird eine Strömung von zuströmender Prüfflüssigkeit im Zwischenraum zwischen den Hohlfasermembranen vom Bereich C in den Bereich B weitgehend unterbunden. Eine Restmenge von Prüfflüssigkeit, die im Zwischenraum zwischen den Fasern in den Bereich B strömt, oder durch Ultrafiltration aus den Lumen im Bereich B in den Zwischenraum zwischen die Hohlfasermembranen dringt ist Leckageflüssigkeit und wird aus dem Gehäuse durch den Flüssigkeitsauslass (409) abgeführt. In manchen Ausführungen ist es je nach Menge der anfallenden Leckageflüssigkeit für die Genauigkeit der Messung erforderlich, dass die Leckageflüssigkeit abgeführt wird. In diesem Fall ist es erforderlich, dass Bereich B von Bereich A durch eine weitere Kompressionsvorrichtung getrennt wird und Bereich B mit einem weiteren Flüssigkeitsauslass (409) kommuniziert. Über den weiteren Flüssigkeitsauslass (409) stellt sich ein Druck im Zwischenraum zwischen den Hohlfasermembranen ein, der dem Umgebungsdruck entspricht. Im Zwischenraum zwischen den Hohlfasermembranen im Bereich C herrscht ein Druck herrscht, der über einen Flüssigkeitsauslass (440) dem Umgebungsdruck entspricht. Der Druck im Zwischenraum zwischen den Hohlfasermembranen im Bereich B und Bereich C ist im Wesentlichen gleich. Über die weitere Kompressionsvorrichtung (450) und/ oder (460) kann sich damit keine Leckageflüssigkeit in den Bereich C fortbewegen. Eine Vorrichtung nach Fig.4 hat sich insbesondere für Hohlfasermembranen mit sehr hohen Ultrafiltrationskoeffizienten als vorteilhaft erwiesen,

## Beispiel 1 - Versuchsdurchführung

Nachfolgend wird das erfindungsgemäße Verfahren anhand eines Beispiels beschrieben, ohne aber auf dieses Beispiel beschränkt zu sein. Das Wasserbad (102) wird mit destilliertem Wasser aufgefüllt und auf 37°C temperiert. Das zu untersuchende Faserbündel wird mit Hilfe einer Aluminiumfolie auf einer Heizplatte bei ca. 300°C auf einer Seite zu geschmolzen. Das so behandelte Hohlfasermembranbündel (220, 320) wird in die Gehäusehülse (201, 301) eingeformt.

Die Gehäusehülse (201, 301) wird nun in das Aufnahmeteil (202, 303) eingebracht. Der Dorn (205) wird zusammen mit der den Dorn umschließenden Hülse (204, 340) bis zum Anschlag in das Faserbündel (220, 320) gedrückt. Anschließend wird der Dorn (205) herausgezogen und die Hülse (340) verbleibt im Bündel.

Eine Luftmanschette wird am Lochkranz des Gaseinlasses (208, 308) angebracht und ein kleiner Luftstrom zugeleitet. Der Lochkranz des Gasauslasses (207,307) wird geschlossen.

Die Pumpe wird eingeschaltet. Nach der Konditionierung, d.h. nach einem Pumpvorgang von je nach Membrantyp von 5 oder 10 Minuten wird das Ultrafiltrat in einem Behältnis aufgefangen und zu definierten Messzeitpunkten gewogen. Der Ultrafiltrationskoeffizient kann unter Zuhilfenahme des gemessenen Transmembrandrucks berechnet werden.

## Beispiel 2 Messprinzip

Die Ultrafiltrationsrate wird direkt am zu untersuchenden Hohlfasermembranbündel gemessen (220, 320), ohne dass dazu das Hohlfasermembranbündel in ein Filtermodul vergossen und eingebaut werden muss. Als Alternative zu dem im Filtermodul vorhandenen Verguss, wird ein sehr starker Strömungswiderstand im Zwischenraum zwischen den Hohlfasermembranen erzeugt. Damit wird eine Strömung im Bereich dieses Strömungswiderstands, der dem komprimierten Teil des Hohlfasermembranbündels in dem Gehäuse entspricht (222, 322), weitgehend unterbunden. Der erzeugte Strömungswiderstand liefert damit die gleiche Wirkung, wie ein sonst üblicher Verguss, indem Flüssigkeitsströmung zwischen den Hohlfasermembranen unterbunden wird, die endseitig offenen Hohlfasermembranen aber zuströmender Flüssigkeit zugänglich bleiben, und so Flüssigkeit in die Lumen des Hohlfasermembranbündels eintreten kann.

Das in das Gehäuse der Messvorrichtung (100, 200, 300) eingeformte Hohlfasermembranbündel (221, 321) wird für die Messdurchführung mit Prüfflüssigkeit durchspült. Die Prüfflüssigkeit wird durch den Flüssigkeitseinlass (212, 312) in die Messvorrichtung (100, 200, 300), insbesondere in das Aufnahmeteil des Gehäuses (203, 303) eingeströmt und der Eingangsdruck auf 200 mbar eingestellt. Der Eingangsdruck kann mittels Druckmesser (105) und Pumpe (103) eingestellt werden.

Der Eingangsdruck stellt sich gemäß den Ausführungsformen aus Fig.1 und Fig. 3 im Aufnahmeteil und auf der Lumenseite der Hohlfasermembranen über die gesamte Länge der Hohlfasermembran ein. Die Prüfflüssigkeit kann nur durch den transmembranen Übergang in den Zwischenraum zwischen den Hohlfasermembranen im Abschnitt des nicht komprimierten Teils (321) des Hohlfasermembranbündels gelangen. Bei diesem Übergang bildet sich ein Druckgefälle aus, so dass im Zwischenraum zwischen den Hohlfasermembranen im nicht komprimierten Teil (321) ein Wasserdruck herrscht, der dem Umgebungsdruck entspricht. Das durch Ultrafiltration anfallende Wasser im Zwischenraum zwischen den Hohlfasermembranen fließt durch den (340) Flüssigkeitsauslass nach außen. Das transmembrane Druckgefälle bedingt dabei die Ultrafiltration.

Die Messung wird umso präziser, je besser die Wasserströme auf den jeweiligen Membranseiten voneinander getrennt werden können. Demzufolge ist es wichtig, dass möglichst das ganze Ultrafiltrat nach innen zum zentralen Flüssigkeitsauslass (304) geleitet wird. Das durch Ultrafiltration in den Zwischenräumen zwischen den Hohlfasermembranen befindliche Wasser wird zum zentralen Flüssigkeitsauslass (304) geleitet. Dies erfolgt durch den Gaseinlass (308), durch den ein Luftstrom in das Gehäuse eingeleitet wird. Der Lochkreis für die Hilfsluft ist dabei so an der Gehäusehülse (302) angebracht, dass die Wassertropfen, die sich im Hohlfasermembranbündel (220, 320) bilden können von außen nach innen zum ersten Ende (341) des Flüssigkeitsauslasses (340) geführt werden und abgeleitet werden.

Für eine starke und reproduzierbare Kompression des Bündels wird ein Abschnitt des eingeformten Hohlfasermembranbündels mittels des Dorns (205) und dem hülsenförmigen Flüssigkeitsauslass (204, 340) so stark deformiert, dass ein maximaler Strömungswiderstand zwischen den Hohlfasermembranen erhalten wird, ohne Hohlfasermembranen zu beschädigen. Durch die Verdrängung der Hohlfasermembranen steigt die Packungsdichte im komprimierten Abschnitt der Hohlfasermembran im vorliegenden Anwendungsbeispiel von ca. 60% auf ca. 95% an.

## Patentansprüche

1. Verfahren zur Bestimmung wenigstens der Ultrafiltrationsrate und/ oder des Ultrafiltrationskoeffizienten, von Hohlfasermembranen, wobei die Ultrafiltrationsrate die Menge der permeierten Flüssigkeit pro Zeiteinheit ist und der Ultrafiltrationskoeffizient der Ultrafiltrationsrate bezogen auf die transmembrane Druckdifferenz entspricht, aufweisend die Schritte:

(a) Bereitstellen eines Hohlfasermembranbündels (220, 320), aufweisend eine Vielzahl von Hohlfasermembranen (420, 421) mit einem ersten Ende und einem zweiten Ende, wobei die Lumen der Hohlfasermembranen an dem ersten Ende des Hohlfasermembranbündels endseitig offen, insbesondere flüssigkeitsdurchlässig und an dem zweiten Ende des Hohlfasermembranbündels endseitig verschlossen, insbesondere flüssigkeitsdicht ausgestaltet sind,

(b) Bereitstellen eines Gehäuses (101, 201, 301, 401) zur Aufnahme des Hohlfasermembranbündels aufweisend ein erstes Ende und ein zweites Ende, wobei das erste Ende mindestens einen Flüssigkeitseinlass (212, 312) aufweist,

(c) Einbringen des Hohlfasermembranbündels (220, 320) in das Gehäuse (101, 201, 301, 401), wobei das erste Ende des Hohlfasermembranbündels (220, 320) mit den endseitig offenen Lumen der Hohlfasermembranen (420, 421) zu dem zumindest einen Flüssigkeitseinlass (212, 312) am ersten Ende des Gehäuses (101, 201, 301, 401) ausgerichtet ist,

(d) Bestimmen einer Permeationseigenschaft der Hohlfasermembranen (420, 421)

wobei, dass das Verfahren weiter die Schritte umfasst:

(e) Bereitstellen von zumindest einer Kompressionsvorrichtung (204, 340, 450, 451, 460, 461) zum komprimieren des Hohlfasermembranbündels in zumindest einem Teilbereich (222, 322) des Hohlfasermembranbündels, insbesondere des Teilbereichs des Hohlfasermembranbündels, welcher sich an das erste Ende des Hohlfasermembranbündels anschließt, um die Packungsdichte der Hohlfasermembranen in dem zumindest einen Teilbereich des Hohlfasermembranbündels zu erhöhen

(f) Bereitstellen von zumindest einem Flüssigkeitsauslass (204, 340, 440),

(g) Bereitstellen von zumindest einer Prüfflüssigkeit,

(h) Zuströmen der zumindest einen Prüfflüssigkeit durch den wenigstens einen Flüssigkeitseinlass (212, 312, 412) am ersten Ende des Gehäuses (101, 201, 301, 401) ins Innere des Gehäuses und/oder des Hohlfasermembranbündels und/ oder der Hohlfasermembranen (420,421),

(i) Komprimieren des Hohlfasermembranbündels in zumindest einem Teilbereich des Hohlfasermembranbündels (222, 322), insbesondere des Teilbereichs des Hohlfasermembranbündels, welcher sich an das erste Ende des Hohlfasermembranbündels anschließt mittels der zumindest einen Kompressionsvorrichtung (203, 340, 450, 451, 460, 461), um die Packungsdichte der Hohlfasermembranen in dem zumindest einen Teilbereich des Hohlfasermembranbündels zu erhöhen

wobei in Schritt

(d) die Ultrafiltrationsrate und/ oder der auf die transmembrane Druckdifferenz bezogene Ultrafiltrationskoeffizient, der Hohlfasermembranen, durch Aufnehmen wenigstens eines Messwertes, insbesondere durch Messung der austretenden Menge der zumindest einen Prüfflüssigkeit, an zumindest einem Flüssigkeitsauslass (204, 340, 440) bestimmt wird,
**dadurch gekennzeichnet, dass**
der komprimierte Teilbereich des Hohlfasermembranbündels (222, 322) so komprimiert ist, dass ein Flüssigkeitsstrom der zuströmenden Prüfflüssigkeit zwischen den Hohlfasermembranen im Wesentlichen unterbunden wird und die Enden des Hohlfasermembranbündels nicht in einem Gießharz vergossen sind.

2. Verfahren nach Anspruch 1, **gekennzeichnet durch** den Schritt:
(j) Mehrfaches Aufnehmen von Messwerten oder kontinuierliches Aufnehmen eines Messwerteverlaufs an zumindest einem Flüssigkeitsauslass (204, 340, 440) während des Zuströmens der wenigstens einen Prüfflüssigkeit in das Gehäuse (101, 210, 301, 401) durch wenigstens einen Flüssigkeitseinlass (212, 312, 412) des Gehäuses.

3. Verfahren nach wenigstens einem der vorhergehenden Ansprüche 1 oder 2, **dadurch gekennzeichnet, dass** die Packungsdichte der Hohlfasermembranen im komprimierten Teilbereich (222, 322) des Hohlfasermembranbündels mehr als 80 % bis 150%, bevorzugt 85% bis 120%, weiter bevorzugt 90% bis 110% beträgt.

4. Verfahren nach zumindest einem der vorhergehenden Ansprüche 1 bis 3, **dadurch gekennzeichnet, dass** die Kompression des Hohlfasermembranbündels durch Einbringen der mindestens einen Kompressionsvorrichtung ins Innere (204, 340, 460, 461) des Hohlfasermembranbündels bewirkt wird und/oder durch Krafteinwirkung auf das Äußere (450,451) des Hohlfasermembranbündels durch die zumindest eine Kompressionsvorrichtung bewirkt wird.

5. Verfahren nach zumindest einem der vorhergehenden Ansprüche 1 bis 4, **dadurch gekennzeichnet, dass** die Packungsdichte der Hohlfasermembranen im nicht komprimierten Teilbereich des Hohlfasermembranbündels (221, 321) weniger als 70 % beträgt.

6. Verfahren nach einem der vorhergehenden Ansprüche 1 bis 5, **dadurch gekennzeichnet, dass** Leckageflüssigkeit im komprimierten Teil des Hohlfasermembranbündels aus dem Gehäuse durch wenigstens einen weiteren Flüssigkeitsauslass (209, 309, 409) am Gehäuse abgeleitet wird.

7. Verfahren nach zumindest einem der vorhergehenden Ansprüche 1 bis 6, **dadurch gekennzeichnet, dass** der zumindest eine Flüssigkeitsauslass (204, 340, 440), Bestandteil des Gehäuses ist oder Bestandteil der zumindest einen Kompressionsvorrichtung (204, 340) ist.

8. Verfahren nach einem der vorhergehenden Ansprüche 1 bis 7, **dadurch gekennzeichnet, dass** die Kompression des Hohlfasermembranbündels im komprimierten Teilbereich durch mindestens zwei Kompressionsvorrichtungen (450, 451, 460, 461) bewirkt wird wobei optional
der wenigstens eine weitere Flüssigkeitsauslass (409) so am Gehäuse angebracht ist, dass auftretende Leckageflüssigkeit, die zwischen den mindestens zwei Kompressionsvorrichtungen entsteht, abgeleitet wird.

9. Verfahren nach zumindest einem der vorhergehenden Ansprüche 1 bis 8, **dadurch gekennzeichnet, dass** weiterhin zumindest ein Gaseinlass (208, 308) und zumindest ein Gasauslass (207, 307) am Gehäuse und/oder der zumindest einen Kompressionsvorrichtung vorliegt, wobei optional
das Gas, insbesondere Luft, durch den zumindest einen Gaseinlass (208, 308) eintritt, wobei das eintretende Gas durch die Zwischenräume zwischen den Hohlfasermembranen des Hohlfasermembranbündels strömt und gegebenenfalls zumindest teilweise durch den zumindest einen Gasauslass wieder aus den Zwischenräumen zwischen den Hohlfasermembranen des Hohlfasermembranbündels austreten kann.

10. Verfahren nach zumindest einem der vorhergehenden Ansprüche 1 bis 9, **dadurch gekennzeichnet, dass** die zumindest eine zuströmende Prüfflüssigkeit mit einem Einlassdruck von 50 mbar bis 500 mbar, bevorzug 100 mbar bis 300 mbar, weiter bevorzugt 150 mbar bis 250 mbar ins Innere des Gehäuses (101, 201, 301) und/oder des Hohlfasermembranbündels (220, 320) zugeleitet wird, wobei optional der Druck der Prüfflüssigkeit in den Lumen der Hohlfasermembranen auf der gesamten Länge der Hohlfasermembranen dem Einlassdruck der zumindest einen Prüfflüssigkeit entspricht.

11. Verfahren gemäß zumindest einem der vorhergehenden Ansprüche 1 bis 10, **dadurch gekennzeichnet, dass** nach dem zumindest ersten Flüssigkeitsauslass (209, 309, 409) die in den Zwischenräumen der Hohlfasermembran vorliegende Prüfflüssigkeit im Wesentlichen Prüfflüssigkeit ist, welche aus den Lumen der Hohlfasermembranen durch die Hohlfasermembran hindurch getreten ist.

12. Vorrichtung zum Durchführen eines Verfahrens nach zumindest einem der Ansprüche 1 bis 11, aufweisend:

(a) zumindest ein Reservoir (102) zur Aufnahme mindestens einer Prüfflüssigkeit,

(b) ein Gehäuse (101) mit einem ersten Ende und einem zweiten Ende zur Aufnahme eines Hohlfasermembranbündels, wobei das erste Ende des Gehäuses zumindest ein Flüssigkeitseinlass (212, 312, 412) aufweist,

(c) zumindest eine Verbindungsvorrichtung, insbesondere Pumpmittel (104), zum Zuführen der zumindest einen Prüfflüssigkeit von dem zumindest ein Reservoir ins Innere des Gehäuses (101, 201, 301) und/oder des Hohlfasermembranbündels (220, 320), bzw. der Hohlfasermembranen.

(d) Zumindest eine Kompressionsvorrichtung (204, 340,) zum Komprimieren von zumindest einem Teilbereich (222, 322) des Hohlfasermembranbündels, um die Packungsdichte der Hohlfasermembranen in dem zumindest einen Teilbereich des Hohlfasermembranbündels zu erhöhen, wobei es sich bei der zumindest einen Kompressionsvorrichtung um einen Dorn und/ oder eine Hülse handelt, welche ins Zentrum des Hohlfasermembranbündels und parallel zur Längsachse des Gehäuses eingebracht werden kann,

(e) zumindest einen Flüssigkeitsauslass (204, 340, 440),

(f) Mittel zur zeitabhängigen Messung der Ultrafiltrationsrate und/ oder des Ultrafiltrationskoeffizient an zumindest einem Flüssigkeitsauslass (204, 340, 440), wobei die Ultrafiltrationsrate die Menge der permeierten Flüssigkeit pro Zeiteinheit ist und der Ultrafiltrationskoeffizient der Ultrafiltrationsrate bezogen auf die transmembrane Druckdifferenz entspricht.

**13.** Vorrichtung nach Anspruch 12, **dadurch gekennzeichnet, dass** die Vorrichtung weiterhin Mittel zum Bestimmen des Drucks am zumindest einen Flüssigkeitseinlass des Gehäuses aufweist oder **dadurch gekennzeichnet, dass** die Vorrichtung zumindest einen Gaseinlass (207, 308) und zumindest einen Gasauslass (208, 308) aufweist.

**14.** Verfahren zur Herstellung von Hohlfasermembran-Filtermodulen aufweisend die Schritte:

(a) Festlegen von einem oder mehreren Wertebereichen für wenigstens eine oder mehrere Permeationseigenschaften, insbesondere Festlegen von einem oder mehreren Wertebereichen für die Ultrafiltrationsrate und/ oder des auf die transmembrane Druckdifferenz bezogenen Ultrafiltrationskoeffizienten, von Hohlfasermembranen, die für die Herstellung von Hohlfasermembran-Filtermodulen vorgesehen sind,

(b) Auswählen von einem oder mehreren Herstellparametern um Hohlfasermembranen mit der wenigstens einen Permeationseigenschaft, oder den mehreren Permeationseigenschaften, mit dem im Schritt (a) festgelegten Wertebereich herzustellen,

(c) Herstellen von Hohlfasermembranen durch ein Spinnverfahren nach einem oder mehreren im Schritt (b) ausgewählten Herstellparametern,

(d) Bündeln der erhaltenen Hohlfasermembranen zu Hohlfasermembranbündeln,

(e) Durchführen eines Verfahrens nach einem der Ansprüche 1 bis 11 zur Bestimmung der Ultrafiltrationsrate und/ oder des Ultrafiltrationskoeffizienter Hohlfasermembranen , wobei die Ultrafiltrationsrate die Menge der permeierten Flüssigkeit pro Zeiteinheit ist und der Ultrafiltrationskoeffizient der Ultrafiltrationsrate bezogen auf die transmembrane Druckdifferenz entspricht,

(f) Verwendung der Hohlfasermembranbündel für den Bau von Hohlfasermembran-Filtermodulen wenn festgestellt wird, dass die einen oder mehreren Permeationseigenschaften im in Schritt (a) festgelegten Wertebereich liegen.

**15.** Verfahren zur Herstellung von Hohlfasermembran-Filtermodulen nach Anspruch 14, **gekennzeichnet durch** die Anpassung des wenigstens einen oder der mehreren ausgewählten Herstellparameter aus Schritt (b), wenn festgestellt wird, dass die eine Permeationseigenschaft oder die mehreren Permeationseigenschaften der hergestellten Hohlfasermembranen nicht im in Schritt (a) festgelegten einen Wertebereich oder den mehreren Wertebereichen liegen, so dass entsprechend der Anpassung der Herstellparameter die Permeationseigenschaften hergestellter Hohlfasermembranen wieder in dem in Schritt (a) festgelegten Wertebereich liegen.

**Claims**

**1.** Method for determining at least the ultrafiltration rate and/or the ultrafiltration coefficient of hollow fiber membranes, wherein the ultrafiltration rate is the amount of permeated liquid per unit time and the ultrafiltration coefficient corresponds to the ultrafiltration rate related to the transmembrane pressure difference, comprising the steps:

(a) providing a hollow fiber membrane bundle (220, 320) comprising a plurality of hollow fiber membranes (420, 421) having a first end and a second end, wherein the lumens of the hollow-fibre membranes at the first end of the hollow-fibre membrane bundle are terminally open, more particularly liquid-permeable, and at the second end of the hollow-fibre membrane bundle are terminally closed, more particularly liquid-tight,

(b) providing a housing (101, 201, 301, 401) for receiving the hollow fiber membrane bundle comprising a first end and a second end, wherein the first end comprises at least one liquid inlet (212, 312),

(c) introducing the hollow-fibre membrane bundle (220, 320) into the housing (101, 201, 301, 401), wherein the first end of the hollow-fibre membrane bundle (220, 320) having the terminally open lumens of the hollow-fibre membranes (420, 421) is oriented towards the at least one liquid inlet (212, 312) at the first end of the housing (101, 201, 301, 401),

(d) determining a permeation property of the hollow fiber membranes (420, 421).

wherein, the method further comprises the steps of:

(e) providing at least one compression apparatus (204, 340, 450, 451, 460, 461) for compressing the hollow fiber membrane bundle in at least a subregion (222, 322) of the hollow fiber membrane bundle, more particularly for compressing the hollow-fibre membrane bundle subregion adjoining the first end of the hollow-fibre membrane bundle, to increase the packing density of the hollow fiber membranes in the at least one portion of the hollow fiber membrane bundle,

(f) providing at least one liquid outlet (204, 340, 440),

(g) providing at least one test liquid,

(h) flowing the at least one test liquid through the at least one liquid inlet (212, 312, 412) at the first end of the housing (101, 201, 301, 401) into the interior of the housing and/or the hollow fiber membrane bundle and/or the hollow fiber membranes (420, 421),

(i) compressing the hollow fiber membrane bundle in at least a subregion of the hollow fiber membrane bundle (222, 322), more particularly compressing the hollow-fibre membrane bundle subregion adjoining the first end of the hollow-fibre membrane bundle by means of the at least one compression apparatus (203, 340, 450, 451, 460, 461) to increase the packing density of the hollow fiber membranes in the at least one subregion of the hollow fiber membrane bundle,

wherein in step

(d) the ultrafiltration rate and/or the ultrafiltration coefficient related to the transmembrane pressure difference, of the hollow fiber membranes is determined by recording at least one measured value, more particularly by measuring the emerging amount of the at least one test liquid at at least one liquid outlet (204, 340, 440) **characterized in that** the compressed subregion of the hollow fiber membrane bundle (222, 322) is in a compressed state such that a liquid stream of the inflowing test liquid between the hollow-fibre membranes is essentially prevented, and

**characterized in that** the ends of the hollow fiber membrane bundle are not encapsulated in a casting resin.

2. The method according to claim 1, **characterized by** the step of:
(j) repeatedly recording measured values or continuously recording a measured-value trajectory at one or more than one liquid outlet (204, 340, 440) during the step of inflowing the at least one test liquid into the housing (101, 210, 301, 401) through at least one liquid inlet (212, 312, 412) of the housing.

3. The method according to at least one of the preceding claims 1 or 2, **characterized in that** the packing density of the hollow fiber membranes in the compressed subregion (222, 322) of the hollow fiber membrane bundle is more than 80% to 150%, preferably 85% to 120%, more preferably 90% to 110%.

4. The method according to at least one of the preceding claims 1 to 3, **characterized in that** the compression of the hollow fiber membrane bundle is effected by introducing the at least one compression apparatus into the interior (204, 340, 460, 461) of the hollow fiber membrane bundle and/or is effected by applying force onto the exterior (450, 451) of the hollow fiber membrane bundle by the at least one compression apparatus.

5. The method according to at least one of the preceding claims 1 to 4, **characterized in that** the packing density of the hollow fiber membranes in the non-compressed subregion of the hollow fiber membrane bundle (221, 321) is less than 70%.

6. The method of any one of the preceding claims 1 to 5, **characterized in that** leakage liquid in the compressed portion of the hollow fiber membrane bundle is drained of from the housing through at least one further liquid outlet (209, 309, 409) on the housing.

7. The method of at least one of the preceding claims 1 to 6, **characterized in that** the at least one liquid outlet (204, 340, 440), is constituent part of the housing or is constituent part of the at least one compression apparatus (204, 340).

8. The method according to any one of the preceding claims 1 to 7, **characterized in that** the compression of the hollow fiber membrane bundle in the compressed subregion is effected by at least two compression apparatus (450, 451, 460, 461) wherein optionally the at least one further liquid outlet (409) is mounted to the housing in such a way that any leakage liquid occurring between the at least two compression apparatuses is drained off.

9. A method according to at least one of the preceding claims 1 to 8, **characterized in that** further at least one gas inlet (208, 308) and at least one gas outlet (207, 307) are present on the housing and/or on the at least one compression apparatus, wherein optionally
the gas, in particular air, enters through the at least one gas inlet (208, 308), wherein the entering gas flows through the interspaces between the hollow fiber membranes of the hollow fiber membrane bundle and, optionally, can at least partially exit again through the at least one gas outlet from the interspaces between the hollow fiber membranes of the hollow fiber membrane bundle.

10. Method according to at least one of the preceding claims 1 to 9, **characterized in that** the at least one inflowing test liquid is fed into the interior of the housing (101, 201, 301) and/or the hollow fiber membrane bundle (220, 320) with an inlet pressure of 50 mbar to 500 mbar, preferably 100 mbar to 300 mbar, further preferably 150 mbar to 250 mbar, wherein optionally
the pressure of the test liquid in the lumens of the hollow fiber membranes over the entire length of the hollow fiber membranes corresponds to the inlet pressure of the at least one test liquid.

11. The method according to at least one of the preceding claims 1 to 10, **characterized in that** downstream the at least first liquid outlet (209, 309, 409), the test liquid present in the interspaces of the hollow fiber membrane is substantially test liquid that has passed through the hollow fiber membrane from the lumens of the hollow fiber membranes.

12. Apparatus for performing a method according to at least one of claims 1 to 11, comprising:

(a) at least one reservoir (102) for holding at least one test liquid,
(b) a housing (101) having a first end and a second end for receiving a hollow fiber membrane bundle, the first end of the housing having at least one liquid inlet (212, 312, 412),
(c) at least one connecting device, in particular pumping means (104), for supplying the at least one test liquid from the at least one reservoir to the interior of the housing (101, 201, 301) and/or the hollow fiber membrane bundle (220, 320), respectively to the hollow fiber membranes,
(d) at least one compression apparatus (204, 340,) for compressing at least a subregion (222, 322) of the hollow fiber membrane bundle to increase the packing density of the hollow fiber membranes in the at least one subregion of the hollow fiber membrane bundle, wherein the at least one compression apparatus is a mandrel and/or sleeve that can be inserted into the center of the hollow fiber membrane bundle and parallel to the longitudinal axis of the housing,
(e) at least one liquid outlet (204, 340, 440)
(f) means for time-dependent measurement of the ultrafiltration rate and/or the ultrafiltration coefficient at at least one liquid outlet (204, 340, 440), wherein the ultrafiltration rate is the amount of permeated liquid per unit time and the ultrafiltration coefficient corresponds to the ultrafiltration rate relative to the transmembrane pressure difference.

13. The apparatus of claim 12, **characterized in that** the apparatus further comprises means for determining the pressure at the at least one liquid inlet of the housing or **characterized in that** the apparatus comprises at least one gas inlet (207, 308) and at least one gas outlet (208, 308).

14. A method of manufacturing hollow fiber membrane filter modules comprising the steps of:

(a) defining one or more value ranges for at least one or more permeation properties, in particular defining one or more value ranges for the ultrafiltration rate and/or the ultrafiltration coefficient related to the transmembrane pressure difference, of hollow fiber membranes intended for the manufacture of hollow fiber membrane filter modules,
(b) selecting one or more manufacturing parameters to produce hollow fiber membranes having the at least one permeation property, or the one or more of permeation properties, within the value range determined in step (a),(a)
(c) producing hollow fiber membranes by a spinning process according to one or more manufacturing parameters selected in step (b),(b)
(d) bundling the obtained hollow fiber membranes into hollow fiber membrane bundles,
(e) performing a method according to any one of claims 1 to 11 for determining the ultrafiltration rate and/or ultrafiltration coefficient of hollow fiber membranes, wherein the ultrafiltration rate is the amount of permeated liquid per unit time and the ultrafiltration coefficient corresponds to the ultrafiltration rate relative to the transmembrane pressure difference,
(f) using the hollow fiber membrane bundles for the manufacture of hollow fiber membrane filter modules once it has been ascertained that the one or more permeation properties are within the value range specified in step (a).(a)

15. The method of manufacturing hollow fiber membrane filter modules of claim 14, wherein the at least one or more selected manufacturing parameters of step (b) are adjusted when it is determined that the one or more permeation properties of the manufactured hollow fiber membranes are not within the one or more value ranges defined in step (a), such that in accordance with the adjustment of the manufacturing parameters, the permeation properties of manufactured hollow fiber membranes are again within the range of values established in step (a).(b)(a)(a)

**Revendications**

1. Procédé de détermination d'au moins un taux d'ultrafiltration et/ou un coefficient d'ultrafiltration, de membranes à fibres creuses, dans lequel le taux d'ultrafiltration est la quantité de liquide obtenu par perméation par unité de temps et le coefficient d'ultrafiltration correspond au taux d'ultrafiltration rapporté à une différence de pression transmembranaire, présentant les étapes de :

(a) fourniture d'un faisceau de membranes à fibres creuses (220, 320), présentant une pluralité de membranes à fibres creuses (420, 421) avec une première extrémité et une seconde extrémité, dans lequel les lumières des membranes à fibres creuses sont conçus ouvertes au niveau de l'extrémité de la première extrémité du faisceau de membranes à fibres creuses, en particulier perméables aux liquides, et fermés au niveau de la seconde extrémité du faisceau de membranes à fibres creuses, en particulier étanches aux liquides,
(b) fourniture d'un logement (101, 201, 301, 401) pour la réception du faisceau de membranes à fibres creuses présentant une première extrémité et une seconde extrémité, dans lequel la première extrémité présente au moins une admission de liquide (212, 312),
(c) insertion du faisceau de membranes à fibres creuses (220, 320) dans le logement (101, 201, 301, 401), dans lequel la première extrémité du faisceau de membranes à fibres creuses (220, 320) est orientée avec les lumières des membranes à fibres creuses (420, 421) ouvertes vers la au moins une admission de liquide (212, 312) au niveau de la première extrémité du logement (101, 201, 301, 401),
(d) détermination d'une perméabilité des membranes à fibres creuses (420, 421)

dans lequel, le procédé comprend en outre les étapes de :

(e) fourniture d'au moins un dispositif de compression (204, 340, 450, 451, 460, 461) pour comprimer le faisceau de membranes à fibres creuses en au moins une zone partielle (222, 322) du faisceau de membranes à fibres creuses, en particulier de la zone partielle du faisceau de membranes à fibres creuses qui se raccorde à la première extrémité du faisceau de membranes à fibres creuses, pour augmenter la densité de tassement des membranes à fibres creuses dans l'au moins une zone partielle du faisceau de membranes à fibres creuses
(f) fourniture d'au moins une évacuation de liquide (204, 340, 440),
(g) fourniture d'au moins un liquide d'essai,
(h) afflux de l'au moins un liquide d'essai à travers l'au moins une admission de liquide (212, 312, 412) au niveau de la première extrémité du logement (101, 201, 301, 401) jusque dans l'intérieur du logement et/ou du faisceau de membranes à fibres creuses et/ou des membranes à fibres creuses (420, 421),
(i) compression du faisceau de membranes à fibres creuses en au moins une zone partielle du faisceau de membranes à fibres creuses (222, 322), en particulier de la zone partielle du faisceau de membranes à fibres creuses qui se raccorde à la première extrémité du faisceau de membranes à fibres creuses au moyen de l'au moins un dispositif de compression (204, 340, 450, 451, 460, 461) pour augmenter la densité de tassement des membranes à fibres creuses dans l'au moins une zone partielle du faisceau de membranes à fibres creuses

dans lequel dans l'étape

(d) le taux d'ultrafiltration et/ou le coefficient d'ultrafiltration rapporté à la différence de pression transmembranaire, des membranes à fibres creuses, est déterminé par enregistrement d'au moins une valeur de mesure, en particulier par mesure de la quantité sortante de l'au moins un liquide d'essai, au niveau d'au moins une évacuation de liquide (204, 340, 440),
**caractérisé en ce que**
la zone partielle comprimée du faisceau de membranes à fibres creuses (222, 322) est comprimée de sorte qu'un écoulement du liquide d'essai affluant est essentiellement empêché entre les membranes à fibres creuses et les extrémités du faisceau de membranes à fibres creuses ne sont pas versées dans une résine coulée.

2. Procédé selon la revendication 1, **caractérisé par** l'étape :
(j) enregistrement à plusieurs reprises de valeurs de mesure ou enregistrement continu d'un déroulé de valeurs de mesure au niveau d'au moins une évacuation de liquide (204, 340, 440) pendant l'afflux du au moins un liquide d'essai jusque dans le logement (101, 201, 301, 401) à travers au moins une admission de liquide (212, 312, 412) du logement.

3. Procédé selon au moins l'une des revendications précédentes 1 ou 2, **caractérisé en ce que** la densité de tassement des membranes à fibres creuses dans la zone partielle (222, 322) comprimée du faisceau de membranes à fibres creuses s'élève à plus de 80 % à 150 %, de préférence 85 % à 120 %, davantage de préférence de 90 % à 110 %.

4. Procédé selon au moins l'une des revendications précédentes 1 à 3, **caractérisé en ce que** la compression du faisceau de membranes à fibres creuses est provoquée par l'insertion de l'au moins un dispositif de compression jusque dans l'intérieur (204, 340, 460, 461) du faisceau de membranes à fibres creuses et/ou par effet de force sur l'extérieur (450, 451) du faisceau de membranes à fibres creuses par le au moins un dispositif de compression.

5. Procédé selon au moins l'une des revendications précédentes 1 à 4, **caractérisé en ce que** la densité de tassement des membranes à fibres creuses dans la zone partielle non comprimée du faisceau de membranes à fibres creuses (221, 321) s'élève à moins de 70 %.

6. Procédé selon l'une des revendications précédentes 1 à 5, **caractérisé en ce qu'**un liquide de fuite dans la partie comprimée du faisceau de membranes à fibres creuses est détourné hors du logement par au moins une autre évacuation de liquide (209, 309, 409) au niveau du logement.

7. Procédé selon au moins l'une des revendications précédentes 1 à 6, **caractérisé en ce que** l'au moins une évacuation de liquide (204, 340, 440) fait partie du logement ou fait partie du au moins un dispositif de compression (204, 340).

8. Procédé selon au moins l'une des revendications précédentes 1 à 7, **caractérisé en ce que** la compression du faisceau de membranes à fibres creuses dans la zone partielle comprimée est provoquée par au moins deux dispositifs de compression (450, 451, 460, 461), dans lequel optionnellement

l'au moins une autre évacuation de liquide (409) est amenée contre le logement de sorte qu'un liquide de fuite apparaissant, qui survient entre les au moins deux dispositifs de compression, soit détourné.

9. Procédé selon au moins l'une des revendications précédentes 1 à 8, **caractérisé en ce qu'**en outre au moins une admission de gaz (208, 308) et au moins une évacuation de gaz (207, 307) sont présentes contre le logement et/ou l'au moins un dispositif de compression, dans lequel optionnellement le gaz, en particulier de l'air, entre à travers l'au moins une admission de gaz (208, 308), dans lequel le gaz entrant s'écoule à travers les espaces intermédiaires entre les membranes à fibres creuses du faisceau de membranes à fibres creuses et le cas échéant peut sortir de nouveau au moins partiellement à travers l'au moins une évacuation de gaz hors des espaces intermédiaires entre les membranes à fibres creuses du faisceau de membranes à fibres creuses.

10. Procédé selon au moins l'une des revendications précédentes 1 à 9, **caractérisé en ce que** l'au moins un liquide d'essai affluant est transmis avec une pression d'admission de 50 mbars à 500 mbars, de préférence 100 mbars à 300 mbars, davantage de préférence de 150 mbars à 250 mbars jusque dans l'intérieur du logement (101, 201, 301) et/ou du faisceau de membranes à fibres creuses (220, 320), dans lequel optionnellement
la pression du liquide d'essai dans les lumières des membranes à fibres creuses correspond sur la longueur totale des membranes à fibres creuses à la pression d'admission de l'au moins un liquide d'essai.

11. Procédé selon au moins l'une des revendications précédentes 1 à 10, **caractérisé en ce qu'**après l'au moins première évacuation de liquide (209, 309, 409) le liquide d'essai présent dans les espaces intermédiaires de la membrane à fibres creuses est essentiellement un liquide d'essai qui est sorti hors des lumières des membranes à fibres creuses à travers la membrane à fibres creuses.

12. Dispositif destiné à exécuter un procédé selon au moins l'une des revendications 1 à 11, présentant :

(a) au moins un réservoir (102) pour la réception d'au moins un liquide d'essai,
(b) un logement (101) avec une première extrémité et une seconde extrémité pour la réception d'un faisceau de membranes à fibres creuses, dans lequel la première extrémité du logement présente au moins une admission de liquide (212, 312, 412),
(c) au moins un dispositif de liaison, en particulier un moyen de pompage (104) pour la conduite de l'au moins un liquide d'essai depuis l'au moins un réservoir jusque dans l'intérieur du logement (101, 201, 301) et/ou du faisceau de membranes à fibres creuses (220, 320) ou des membranes à fibres creuses,

(d) au moins un dispositif de compression (204, 340) pour la compression d'au moins une zone partielle (222, 322) du faisceau de membranes à fibres creuses afin d'augmenter la densité de tassement des membranes à fibres creuses dans l'au moins une zone partielle du faisceau de membranes à fibres creuses, dans lequel l'au moins un dispositif de compression est un mandrin et/ou une douille, qui peut être amené(e) jusque dans le centre du faisceau de membranes à fibres creuses, parallèlement à l'axe longitudinal du logement,

(e) au moins une évacuation de liquide (204, 340, 440),

(f) un moyen pour la mesure en fonction du temps du taux d'ultrafiltration et/ou du coefficient d'ultrafiltration au niveau d'au moins une évacuation de liquide (204, 340, 440), dans lequel le taux d'ultrafiltration est la quantité de liquide obtenu par perméation par unité de temps et le coefficient d'ultrafiltration correspond au taux d'ultrafiltration rapporté à la différence de pression transmembranaire.

13. Dispositif selon la revendication 12, **caractérisé en ce que** le dispositif présente en outre un moyen pour la détermination de la pression au niveau d'au moins une admission de liquide du logement ou **caractérisé en ce que** le dispositif présente au moins une admission de gaz (207, 308) et au moins une évacuation de gaz (208, 308).

14. Procédé de fabrication de modules filtrants à membranes à fibres creuses présentant les étapes de :

(a) fixation d'une ou plusieurs plages de valeurs pour au moins une ou plusieurs perméabilités, en particulier fixation d'une ou plusieurs plages de valeurs pour le taux d'ultrafiltration et/ou du coefficient d'ultrafiltration rapporté à la différence de pression transmembranaire, de membranes à fibres creuses, qui sont prévues pour la fabrication de modules filtrants à membranes à fibres creuses,

(b) sélection d'un ou plusieurs paramètres de fabrication pour fabriquer des membranes à fibres creuses avec l'au moins une perméabilité, ou les plusieurs perméabilités, avec la plage de valeurs fixée à l'étape (a),

(c) fabrication de membranes à fibres creuses par un procédé de filage selon un ou plusieurs paramètres de fabrication sélectionnés à l'étape (b),

(d) assemblage des membranes à fibres creuses obtenues pour constituer des faisceaux de membranes à fibres creuses,

(e) exécution d'un procédé selon l'une des revendications 1 à 11 pour la détermination du taux d'ultrafiltration et/ou du coefficient d'ultrafiltration des membranes à fibres creuses, dans lequel le taux d'ultrafiltration est la quantité de liquide obtenu par perméation par unité de temps et le coefficient d'ultrafiltration correspond au taux d'ultrafiltration rapporté à la différence de pression transmembranaire,

(f) utilisation des faisceaux de membranes à fibres creuses pour la construction de modules filtrants à membranes à fibres creuses lorsqu'il est constaté que les une ou plusieurs perméabilités se trouvent dans la plage de valeurs fixée à l'étape (a).

15. Procédé de fabrication de modules filtrants à membranes à fibres creuses selon la revendication 14, **caractérisé par** l'ajustement de l'au moins un ou des plusieurs paramètres de fabrication sélectionnés à l'étape (b), lorsqu'il est constaté que la perméabilité ou les perméabilités des membranes à fibres creuses fabriquées ne se trouvent pas dans la plage de valeurs ou les plages de valeurs fixées à l'étape (a), de sorte qu'en fonction de l'ajustement des paramètres de fabrication les perméabilités de membranes à fibres creuses fabriquées se trouvent de nouveau dans la plage de valeurs fixée à l'étape (a) .

Fig. 1

Fig. 2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102006057101 A1
- WO 2013034611 A
- WO 2013034611 A1
- JP H04150926 B
- EP 0958852 A1
- JP 2007216175 B
- US 4678573 A
- WO 2015014934 A1
- DE 102013203082 A1
- DE 2418012 A1
- DE 10007327 A1

**In der Beschreibung aufgeführte Nicht-Patentliteratur**

- **UHLENBUSCH-KÖRWER ; BONNIE- SCHORN ; GRASSMANN, VIENKEN.** Understanding Membranes and Dialysers. Pabst Science Publishers, 2004